(19) Europäisches Patentamt
European Patent Office
Office européen des brevets

(11) EP 4 527 887 A1

(12) EUROPEAN PATENT APPLICATION
published in accordance with Art. 153(4) EPC

(43) Date of publication:
26.03.2025 Bulletin 2025/13

(21) Application number: 23807259.9

(22) Date of filing: 10.03.2023

(51) International Patent Classification (IPC):
C08L 33/08 (2006.01) C08F 220/06 (2006.01)
C08F 220/18 (2006.01) C08K 5/29 (2006.01)
C08K 5/41 (2006.01)

(52) Cooperative Patent Classification (CPC):
C08F 220/06; C08F 220/18; C08K 5/29; C08K 5/41;
C08L 33/08

(86) International application number:
PCT/JP2023/009304

(87) International publication number:
WO 2023/223643 (23.11.2023 Gazette 2023/47)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 19.05.2022 JP 2022082131

(71) Applicant: Unimatec Co., Ltd.
Tokyo 105-0012 (JP)

(72) Inventor: SAITO, Satoru
Kitaibaraki-shi, Ibaraki 319-1593 (JP)

(74) Representative: Kraus & Lederer PartGmbB
Thomas-Wimmer-Ring 15
80539 München (DE)

# (54) CROSSLINKABLE ACRYLIC RUBBER COMPOSITION

(57) A crosslinkable acrylic rubber composition comprising:
(A) An acrylic elastomer copolymer comprising a copolymerizable antioxidant represented by general formula [I]:

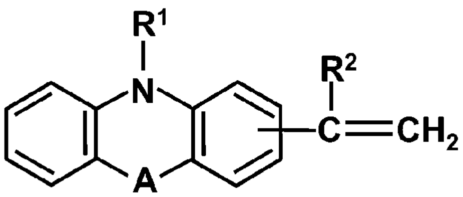

[I]

($R^1$: a monovalent aliphatic hydrocarbon group having 1 to 20 carbon atoms, $R^2$: a hydrogen atom or a methyl group, A is a direct bond, an oxygen atom or a sulfur atom), an alkyl (meth)acrylate monomer and/or an alkoxyalkyl (meth)acrylate monomer, and an $\alpha,\beta$-unsaturated carboxylic acid monomer;
(B) a phenothiazine-based antioxidant;
(C) a polyvalent amine crosslinking agent; and
(D) a crosslinking accelerator.

EP 4 527 887 A1

## Description

TECHNICAL FIELD

The present invention relates to a crosslinkable acrylic rubber composition. More particularly, the present invention relates to a crosslinkable acrylic rubber composition that can minimize the decrease in the mechanical properties of a crosslinked product due to thermal oxidative degradation.

BACKGROUND ART

From the perspective of global climate change countermeasures and efficient use of energy, emission regulations for carbon dioxide and NOx gases emitted from internal combustion engines, typified by automobile engines, tend to be more stringent. As a countermeasure thereof, automobile engines are required to have higher output, higher thermal efficiency, and lower and harmless emissions. This tends to increase the temperature in the engine compartment. Along with this trend, polymer materials, such as rubber and plastics, used in the surrounding area are required to have further improved heat resistance.

As specific examples, vehicles equipped with a turbocharger system for the purpose of improving the fuel efficiency of the engine are becoming widespread. Since air guided from the turbocharger to the intercooler and engine has high temperature and high pressure, high heat resistance is required for rubber hose materials that transport the air.

Thus, with the demand for higher temperatures and longer life for polymer materials used in automobile engines, as countermeasures thereof, for example, efforts are being made to improve the heat resistance of the raw material rubber itself of rubber product parts, and appropriate antioxidants are being added to rubber product parts.

As typical antioxidants of rubber members, phenol-based antioxidants and amine-based antioxidants are used. In particular, amine-based antioxidants are used for rubber members used in higher temperature environments.

For example, in the case of acrylic rubber, amine-based antioxidants typified by 4,4'-bis($\alpha$,$\alpha$-dimethylbenzyl) diphenylamine are used as antioxidants (Patent Documents 1 to 4).

Further, as an effort to improve the heat resistance of the acrylic rubber itself, the crosslinking site monomer is changed from an active chlorine-containing unsaturated monomer to an $\alpha$,$\beta$-unsaturated carboxylic acid monomer, thereby forming a strong crosslinking structure that can withstand use in high temperature environments.

However, even improvement the heat resistance of the raw material rubber itself, and amine-based antioxidants cannot fully satisfy the recent heat resistance requirements.

Patent Document 5 states phenothiazine-based antioxidants are effective as antioxidants for rubber materials.

As a rubber material that has excellent vulcanization characteristics, mechanical characteristics, and heat aging characteristics, and that is particularly suitable for use in antivibration rubber, this reference discloses one comprising (A) a diene-based rubber, (B) a bismaleimide compound, and (C) the following phenothiazine compound:

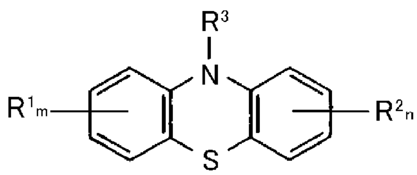

$R^1$, $R^2$: a hydrogen atom, a $C_1$-$C_8$ alkyl group that may be substituted with an aromatic ring, an alkoxy group, a halogen atom, or a cyano group

$R^3$: a hydrogen atom, a $C_1$-$C_6$ chain or cyclic alkyl group, a vinyl group, or an aromatic group

m, n: 0 to 2

A phenothiazine compound in which the sulfur atom at position 5 is $-SO_2-$ is also known and described, for example, in Patent Document 6.

Patent Document 6 discloses a condensed heterocyclic compound represented by the following general formula and an organic material composition comprising the same, and states that it is possible to impart high processing stability, heat resistance, and long life to organic materials such as polymer that is susceptible to oxidative, thermal, or photo-induced breakdown.

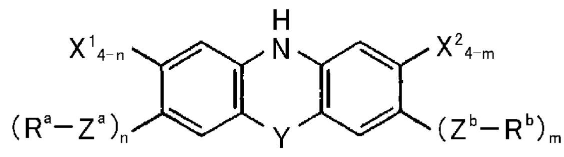

Y: a chemical single bond, -S(=O)-, or -$SO_2$-
$R^a$, $R^b$: a $C_1$- $C_{30}$ organic group that may have a substituent
$Z^a$, $Z^b$: a chemical single bond or -$SO_2$-
$X^1$, $X^2$: a hydrogen atom, a halogen atom, an alkyl group, a cyano group, a nitro group, -$OR^1$, -O-CO-$R^1$, -CO-$OR^1$, -O-CO-$OR^1$, -$NR^2R^3$, -$NR^2$-CO-$R^1$, -CO-$NR^2R^3$, or -O-CO-$NR^2R^3$
n, m: 0 to 2, provided that one of them is not 0.

Moreover, in order to prevent the volatilization of amine-based antioxidants from rubber members, studies have been made on increasing the molecular weight and melting point of amine-based antioxidants. However, along with the increase in the molecular weight and melting point of antioxidants, there are problems such as reducing dispersibility in rubber and transferability inside rubber.
For the purpose of preventing the volatilization of antioxidants and extending the life of rubber parts in high temperature environments, a method of copolymerizing raw material rubber with an antioxidant having a polymerizable unsaturated group has also been examined (Patent Document 7).
For example, in Non-Patent Documents 1 and 2, Nocrac G-1 (produced by Ouchi Shinko Chemical Industrial Co., Ltd.) and APMA (produced by Seiko Chemical Co., Ltd.) are exemplified as antioxidants having a polymerizable unsaturated group.

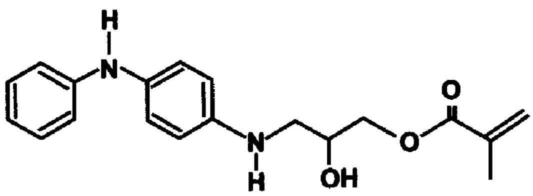

Nocrac G-1

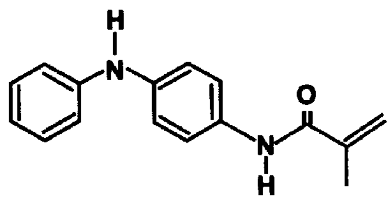

APMA

However, with the above antioxidants, radical copolymerization with a polymerizable unsaturated monomer is practically difficult due to the radical polymerization inhibitory effect of the diphenylamino group.
Moreover, several methods are disclosed for introducing a diphenylamino structure into a polymer by the modification reaction of elastomeric polymer. For example, the following methods are known: a method in which a diphenylamino group is introduced after hydroformylation of the side chain of an elastomer having an olefine-based unsaturated group (Patent Document 8), and a method in which a diphenylamino group is introduced after maleic anhydride is added to a diene-based copolymer in the presence of a free radical generator (Patent Document 9). However, these methods further require a modification step of introducing a diphenylamino group after producing a base copolymer, which is not practical in terms of production cost.
Further, a method of crosslinking acrylic rubber in coexistence with 4-aminodiphenylamine is known (Patent Documents 10 and 11); however, there is concern for this method that 4-aminodiphenylamine may deteriorate compression set resistance characteristics.
As described above, in conventional technologies, none of the methods of improvement in the heat resistance of the raw material rubber itself, improvement in the performance of various antioxidants, and chemical bonding of thermal antioxidant components to the raw material rubber can fully satisfy the recent heat resistance requirements.

PRIOR ART DOCUMENT

PATENT DOCUMENT

Patent Document 1 : JP-A-11-21411
Patent Document 2 :WO 2011/58918 A1

Patent Document 3 : JP-A-2010-254579
Patent Document 4 :WO 2006/001299 A1
Patent Document 5 : JP-A-2015-227402
Patent Document 6 :WO 2011/093443 A1
Patent Document 7 : JP-A-2009-209268
Patent Document 8 : JP-A-4-264106
Patent Document 9 : JP-A-5-230132
Patent Document 10 :WO 2020/158132 A1
Patent Document 11 : JP-A-2009-84514
Patent Document 12 : JP-A-2020-111552

NON-PATENT DOCUMENTS

Non-Patent Document 1 : Rubber Chem.Technol., Vol. 46, pp. 106 (1973)
Non-Patent Document 2 : Rubber Chem.Technol., Vol. 52, pp. 883 (1979)

To address the above problems, the present inventors examined whether a crosslinkable acrylic rubber composition comprising acrylic rubber containing an antioxidant component and a carboxyl group, a phenothiazine-based antioxidant, crosslinking agent and a crosslinking accelerator can be used to improve the heat resistance of the acrylic rubber. In particular, the examination was carried out for the purpose of suppressing significant softening degradation of acrylic rubber comprising ethyl acrylate as a main raw material observed in the early stage of thermal oxidative degradation, and significant hardening degradation observed in the later stage thereof.

OUTLINE OF THE INVENTION

PROBLEM TO BE SOLVED BY THE INVENTION

The present invention was made in view of the problems described above, and an object thereof is to provide a crosslinkable acrylic rubber composition that can suppress significant softening degradation of an acrylic rubber crosslinked product observed in the early stage of thermal oxidative degradation and significant hardening degradation observed in the later stage thereof, and that can minimize the decrease in its mechanical strength.

MEANS FOR SOLVING THE PROBLEM

The above object of the present invention can be achieved by a crosslinkable acrylic rubber composition comprising:

(A) an acrylic elastomer copolymer comprising a copolymerizable antioxidant represented by general formula [I]:

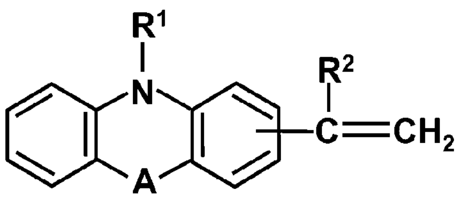

[I]

(wherein $R^1$ is a monovalent aliphatic hydrocarbon group having 1 to 20 carbon atoms, $R^2$ is a hydrogen atom or a methyl group, and A is a direct bond, an oxygen atom or a sulfur atom), an alkyl (meth)acrylate monomer and/or an alkoxyalkyl (meth)acrylate monomer, and an $\alpha,\beta$-unsaturated carboxylic acid monomer;
(B) a phenothiazine-based antioxidant represented by the general formula [II]:

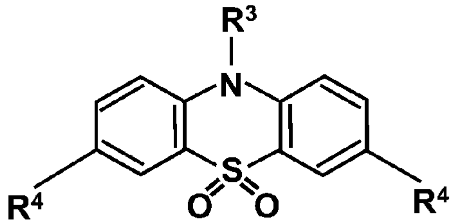

[II]

[wherein $R^3$ is a hydrogen atom, a monovalent aliphatic hydrocarbon group having 1 to 20 carbon atoms, an aralkyl group having 7 to 20 carbon atoms, or an acyl group represented by the following general formula [III]:

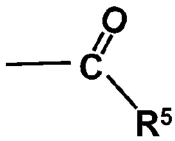

[III]

(wherein $R^5$ is a monovalent aliphatic hydrocarbon group having 1 to 20 carbon atoms), and $R^4$ is an aralkyl group having 7 to 20 carbon atoms];
(C) a polyvalent amine crosslinking agent; and
(D) a crosslinking accelerator.

EFFECT OF THE INVENTION

The acrylic elastomer copolymer used in the crosslinkable acrylic rubber composition of the present invention contains an antioxidant component in the polymer side chain, and the copolymer itself is stabilized against thermal oxidative degradation. Further, in the molded member obtained by crosslinking the crosslinkable acrylic rubber composition, volatilization of the antioxidant component into the air or extraction of the antioxidant component with liquid media, such as fats, oils, and organic solvents, can be prevented. As a result, the acrylic elastomer molded member has characteristics that make its longer life possible under a variety of degrading environments.

Moreover, due to the crosslinking effect of the antioxidant component chemically bonded to the polymer side chain, softening degradation observed in the early stage of thermal oxidative degradation can be suppressed, and the decrease in mechanical strength can be suppressed.

Further, the phenothiazine-based antioxidant that constitutes the composition of the present invention has an effect of suppressing significant hardening degradation in the latter half of thermal oxidative degradation of the acrylic rubber crosslinked product copolymerized with a copolymerizable antioxidant. As a result, the molded member obtained by crosslinking the crosslinkable acrylic rubber composition of the present invention exhibits an excellent effect of minimizing the decrease in mechanical properties throughout the process of thermal oxidative degradation.

BRIEF DESCRIPTION OF DRAWINGS

Fig. 1: A schematic diagram of the 100% modulus change over time of acrylic rubber crosslinked product at 190°C (Example 1: **-●-,** Comparative Example 1: --●--, Comparative Example 2: --▲--, Comparative Example 3: --♦--; Figs 1 to 3 common).
Fig. 2: a schematic diagram of the strength at break change over time of acrylic rubber crosslinked product at 190°C.
Fig. 3: a schematic diagram of the elongation at break change over time of acrylic rubber crosslinked product at 190°C.
Fig. 4: A comparison between the rate of change in strength at break after a combined test composed of air heating (175°C, 150 hours), IRM 903 oil dipping (150°C, 168 hours), and air heating (190°C, 300 hours) was performed (black bar), and the rate of change in strength at break after an air heating aging test (190°C, 300 hours) (white bar).
Fig. 5: A comparison between the rate of change in elongation at break after a combined test composed of air heating (175°C, 150 hours), IRM 903 oil dipping (150°C, 168 hours), and air heating (190°C, 300 hours) was performed (black bar), and the rate of change in elongation at break after an air heating aging test (190°C, 300 hours) (white bar).
Fig. 6: A schematic diagram of the 100% modulus change over time of acrylic rubber crosslinked product at 190°C (Example 2: **-●-,** Comparative Example 4: --●--, Comparative Example 5: **--▲--,** Comparative Example 6: --♦--; Figs 6 to 8 common).
Fig. 7: A schematic diagram of the strength at break change over time of acrylic rubber crosslinked product at 190°C.

Fig. 8: A schematic diagram of the elongation at break change over time of acrylic rubber crosslinked product at 190°C.
Fig. 9: A schematic diagram of the 100% modulus change over time of acrylic rubber crosslinked product at 190°C (Example 3: **-●-,** Comparative Example 7: --●--, Comparative Example 8: **--▲--,** Comparative Example 9: --♦--; Figs 9 to 11 common).
Fig. 10: A schematic diagram of the strength at break change over time of acrylic rubber crosslinked product at 190°C.
Fig. 11: A schematic diagram of the elongation at break change over time of acrylic rubber crosslinked product at 190°C.
Fig. 12: A schematic diagram of the 100% modulus change over time of acrylic rubber crosslinked product at 190°C (Example 4: **-●-,** Comparative Example 10: --●--, Comparative Example 11: **--▲--,** Comparative Example 12: --♦--; Figs 12 to 14 common).
Fig. 13: A schematic diagram of the strength at break change over time of acrylic rubber crosslinked product at 190°C.
Fig. 14: A schematic diagram of the elongation at break change over time of acrylic rubber crosslinked product at 190°C.
Fig. 15: A comparison between the rate of change in elongation at break after a combined test composed of toluene dipping (room temperature, 168 hours), and air heating (190°C, 300 hours) was performed (black bar), and the rate of change in elongation at break after an air heating aging test (190°C, 300 hours) (white bar).

EMBODIMENTS FOR CARRYING OUT THE INVENTION

The crosslinkable acrylic rubber composition of the present invention is constituted of comprising

(A) an acrylic elastomer copolymer comprising a copolymerizable antioxidant represented by general formula [I]:

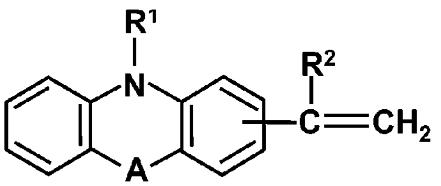

[I]

(wherein $R^1$ is a monovalent aliphatic hydrocarbon group having 1 to 20 carbon atoms, $R^2$ is a hydrogen atom or a methyl group, and A is a direct bond, an oxygen atom or a sulfur atom), an alkyl (meth)acrylate monomer and/or an alkoxyalkyl (meth)acrylate monomer, and an $\alpha,\beta$-unsaturated carboxylic acid monomer;
(B) a phenothiazine-based antioxidant represented by the general formula [II]:

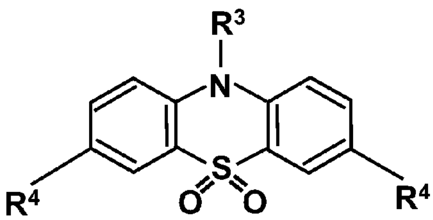

[II]

[wherein $R^3$ is a hydrogen atom, a monovalent aliphatic hydrocarbon group having 1 to 20 carbon atoms, an aralkyl group having 7 to 20 carbon atoms, or an acyl group represented by the following general formula [III]:

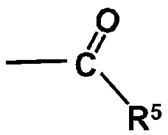

[III]

(wherein $R^5$ is a monovalent aliphatic hydrocarbon group having 1 to 20 carbon atoms), and $R^4$ is an aralkyl group having 7 to 20 carbon atoms];
(C) a polyvalent amine crosslinking agent; and
(D) a crosslinking accelerator.

Here, the term (meth)acrylate refers to acrylate or methacrylate.
The copolymerizable antioxidant represented by the general formula [I] used in the acrylic elastomer copolymer

as the component (A) can be easily produced from phenothiazine, phenoxazine, carbazole, or the like. An example of the production method is shown below.

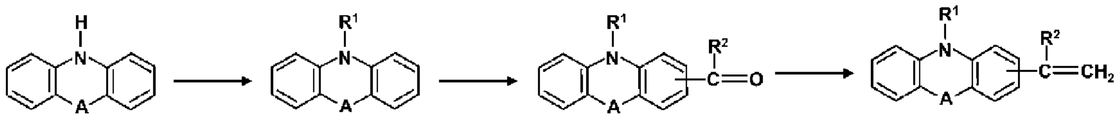

After N-alkylation in the first step, the aromatic ring is formylated or acetylated, and the carbonyl group is then converted to an olefin, whereby the target copolymerizable antioxidant can be produced.

Specific examples of the copolymerizable antioxidant include

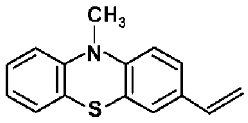
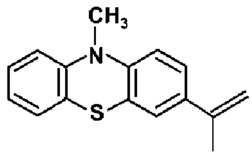
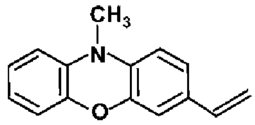
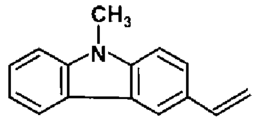

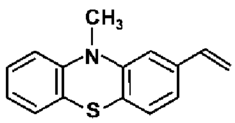
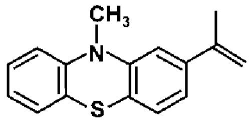
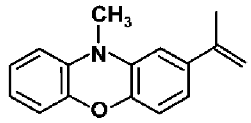
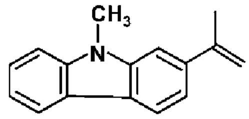

, and the like.

In the copolymerization of a copolymerizable antioxidant and a polymerizable unsaturated monomer, the copolymerizable antioxidant [I] is used at a ratio of about 0.05 to 5 parts by weight, preferably about 0.1 to 3 parts by weight, based on 100 parts by weight of the alkyl (meth)acrylate and/or alkoxyalkyl (meth)acrylate monomer. If the copolymerizable antioxidant is used at a ratio less than this range, a sufficient anti-aging effect cannot be expected. In contrast, even if the copolymerizable antioxidant is used at a ratio greater than this range, no improvement in the anti-aging effect is expected, and it is uneconomical.

As the alkyl (meth)acrylate monomer and/or alkoxyalkyl (meth)acrylate monomer that constitutes the acrylic elastomer copolymer of the present invention, at least one (meth)acrylate selected from alkyl (meth)acrylate containing an alkyl group having 1 to 8 carbon atoms, aralkyl (meth)acrylate containing an aralkyl group having 7 to 20 carbon atoms, and alkoxyalkyl (meth)acrylate containing an alkoxyalkyl group having 2 to 8 carbon atoms is used.

Examples of alkyl (meth)acrylate include methyl (meth)acrylate, ethyl (meth)acrylate, propyl (meth)acrylate, isopropyl (meth)acrylate, n-butyl (meth)acrylate, n-hexyl (meth)acrylate, 2-ethylhexyl (meth)acrylate, n-octyl (meth) acrylate, cyclohexyl (meth)acrylate, and the like. These can be used in combination of two or more. Preferably, ethyl acrylate or two alkyl acrylates, i.e., ethyl acrylate and butyl acrylate, are used.

Examples of aralkyl (meth)acrylate include benzyl (meth)acrylate and the like.

Moreover, examples of alkoxyalkyl (meth)acrylate include methoxymethyl (meth)acrylate, methoxyethyl (meth) acrylate, ethoxyethyl (meth)acrylate, n-butoxyethyl (meth)acrylate, ethoxypropyl (meth)acrylate, methoxyethoxyethyl (meth)acrylate, ethoxyethoxyethyl (meth)acrylate and the like.

Examples of the $\alpha,\beta$-unsaturated carboxylic acid monomer that constitutes the acrylic elastomer copolymer as the component (A) include monobasic $\alpha,\beta$-unsaturated carboxylic acids, dibasic $\alpha,\beta$-unsaturated carboxylic acids, or dibasic $\alpha,\beta$-unsaturated carboxylic acid monoalkyl esters.

Examples of monobasic $\alpha,\beta$-unsaturated carboxylic acid include acrylic acid, methacrylic acid and the like.

Examples of dibasic $\alpha,\beta$-unsaturated carboxylic acid include maleic acid, fumaric acid, itaconic acid, citraconic acid and the like.

Examples of dibasic $\alpha,\beta$-unsaturated carboxylic acid monoalkyl ester include monoalkyl esters of maleic acid, fumaric acid, itaconic acid, and citraconic acid. Specific examples include monomethyl maleate, monoethyl maleate, mono n-propyl maleate, monoisopropyl maleate, mono n-butyl maleate, monoisobutyl maleate, mono n-hexyl maleate, monocyclohexyl maleate, monomethyl fumarate, monoethyl fumarate, mono n-propyl fumarate, monoisopropyl fumarate, mono n-butyl fumarate, monoisobutyl fumarate, mono n-hexyl fumarate, monocyclohexyl fumarate, and the like.

In the acrylic elastomer copolymer as the component (A), the crosslinking site monomer is copolymerized at a ratio of 0.1 to 5 wt.%, preferably 0.5 to 3 wt.%.

Moreover, in addition to these main components of the acrylic elastomer copolymer as the component (A), other polymerizable unsaturated monomers can be used, if necessary.

Examples of polymerizable unsaturated monomer include styrene, $\alpha$-methylstyrene, 3-methylstyrene, 4-methylstyrene, 1-vinylnaphthalene, 2-vinylnaphthalene, acrylonitrile, methacrylonitrile, acrylic acid amide, vinyl acetate, methyl vinyl ether, ethyl vinyl ether, ethylene, propylene, piperylene, butadiene, isoprene, chloroprene, cyclopentadiene, vinyl chloride, vinylidene chloride, and the like.

The acrylic elastomer copolymer as the component (A) is produced by a general method for copolymerizing acrylic rubber. The copolymerization reaction can be carried out by any method, such as an emulsion polymerization method, a suspension polymerization method, a solution polymerization method, or a bulk polymerization method. Preferably, an emulsion polymerization method or a suspension polymerization method is used, and the reaction is carried out at a temperature of about -10 to 100°C, preferably about 5 to 80°C.

Examples of the polymerization initiator for the reaction include organic peroxides or hydroperoxides, such as benzoyl peroxide, dicumyl peroxide, tert-butyl hydroperoxide, cumyl hydroperoxide and p-methylene hydroperoxide; diazo compounds, such as azobisisobutyronitrile and azobisisobutylamidine; ammonium salts represented by ammonium persulfate; peroxide salts, such as sodium salts and potassium salts; and the like. These are used singly or as a redox system.

As an emulsifier used in the particularly preferable emulsion polymerization method, an anionic or nonionic surfactant is used as an aqueous solution or the like whose pH is optionally adjusted by acid or base, and which is formed into a buffer solution by using an inorganic salt.

The polymerization reaction is continued until the conversion rate of the monomer mixture reaches 90% or more. The obtained aqueous latex is coagulated by a salt-acid coagulation method, a method using a salt, such as calcium chloride, magnesium sulfate, sodium sulfate, or ammonium sulfate and the like, a method using a boron compound, such as boric acid or borax, a coagulation method by heat, a freeze coagulation method, or the like. The obtained copolymer is sufficiently washed with water and dried. This acrylic rubber has Mooney viscosity $PML_{1+4}$ (100°C) of about 5 to 100, preferably about 20 to 80.

The acrylic elastomer copolymer as the component (A) is one in which 0.1 to 5 wt.% of a copolymerizable antioxidant represented by the general formula [I], 90 to 99.8 wt.% of an alkyl (meth)acrylate and/or alkoxyalkyl (meth) acrylate monomer, and 0.1 to 5 wt.% of an $\alpha,\beta$-unsaturated carboxylic acid monomer are copolymerized, preferably one in which 0.3 to 3 wt.% of a copolymerizable antioxidant represented by the general formula [I], 94 to 99.2 wt.% of an alkyl (meth)acrylate and/or alkoxyalkyl (meth)acrylate monomer, and 0.5 to 3 wt.% of a crosslinking site monomer are copolymerized. Preferably, an acrylic elastomer copolymer composed of a copolymerizable antioxidant represented by the general formula [I], an alkyl acrylate monomer, and an $\alpha,\beta$-unsaturated carboxylic acid monomer is used.

As the component (B), which is a constituent component of the composition of the present invention, a phenothiazine-based antioxidant represented by the general formula [II] is used.

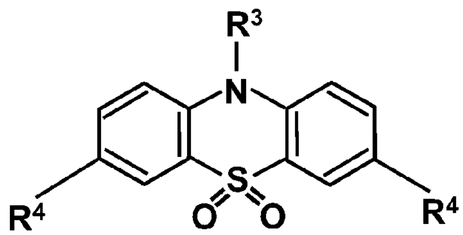

[II]

[wherein $R^3$ is a hydrogen atom, a monovalent aliphatic hydrocarbon group having 1 to 20 carbon atoms, an aralkyl group having 7 to 20 carbon atoms, or an acyl group represented by the following general formula [III]:

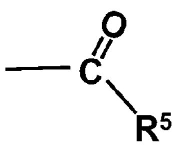

[III]

(wherein $R^5$ is a monovalent aliphatic hydrocarbon group having 1 to 20 carbon atoms), and $R^4$ is an aralkyl group having 7 to 20 carbon atoms].

Specific examples wherein $R^3$ is an aliphatic hydrocarbon group having 1 to 20 carbon atoms include a methyl group, an ethyl group, an n-propyl group, an n-butyl group, an n-pentyl group, an n-hexyl group, an n-heptyl group, an n-octyl group, an n-nonyl group, an n-undecyl group, an n-pentadecyl group, an n-heptadecyl group, an isopropyl group, a 2-butyl group, a 2-pentyl group, a 3-pentyl group, a 2-hexyl group, a 3-hexyl group, a 2-heptyl group, a 3-heptyl group, a 4-heptyl group, a 2-octyl group, a 3-octyl group, a 4-octyl group, a tertiary butyl group, a 1,1-dimethyl-1-propyl group, a 1,1-dimethyl-1-butyl group, a 1,1-dimethyl-1-pentyl group, a 1,1-dimethyl-1-hexyl group, a 3-methyl-3-pentyl group, a 3-ethyl-3-pentyl group, a 3-methyl-3-hexyl group, a 2-ethylhexyl group, a cyclopropyl group, a cyclobutyl group, a

cyclopentyl group, a cyclohexyl group, a cycloheptyl group, a 1-methyl-1-cyclopentyl group, a 1-methyl-1-cyclohexyl group, a 1-adamantyl group, and the like.

Specific examples wherein aralkyl group having 7 to 20 carbon atoms include a benzyl group, an α-methylbenzyl group, a 9-fluorenylmethyl group, and the like.

$R^5$ is a monovalent aliphatic hydrocarbon group having 1 to 20 carbon atoms. Examples of the aliphatic hydrocarbon group having 1 to 20 carbon atoms include a methyl group, an ethyl group, an n-propyl group, an n-butyl group, an n-pentyl group, an n-hexyl group, an n-heptyl group, an n-octyl group, an n-nonyl group, an n-undecyl group, an n-pentadecyl group, an n-heptadecyl group, an isopropyl group, a 2-butyl group, a 2-pentyl group, a 3-pentyl group, a 2-hexyl group, a 3-hexyl group, a 2-heptyl group, a 3-heptyl group, a 4-heptyl group, a 2-octyl group, a 3-octyl group, a 4-octyl group, a tertiary butyl group, a 1,1-dimethyl-1-propyl group, a 1,1-dimethyl-1-butyl group, a 1,1-dimethyl-1-pentyl group, a 1,1-dimethyl-1-hexyl group, a 3-methyl-3-pentyl group, a 3-ethyl-3-pentyl group, a 3-methyl-3-hexyl group, a 2-ethylhexyl group, a cyclopropyl group, a cyclobutyl group, a cyclopentyl group, a cyclohexyl group, a cycloheptyl group, a 1-methyl-1-cyclopentyl group, a 1-methyl-1-cyclohexyl group, a 1-adamantyl group and the like.

In particular, monovalent aliphatic hydrocarbon groups having 4 to 20 carbon atoms, in which the carbon in the α-position with respect to the carbonyl group is tertiary carbon, is preferred. Examples of preferred groups include a tertiary butyl group, a 1,1-dimethyl propyl group, a 1,1-dimethyl-1-butyl group, a 1,1-dimethyl-1-pentyl group, a 1,1-dimethyl-1-hexyl group, a 3-methyl-3-pentyl group, a 3-ethyl-3-pentyl group, a 3-methyl-3-hexyl group, a 2-ethylhexyl group, a 1-methyl-1-cyclopentyl group, a 1-methyl-1-cyclohexyl group, a 1-adamantyl group, and the like.

Examples of the aralkyl group represented by $R^4$ include a benzyl group, an α-methylbenzyl group and an α,α-dimethylbenzyl group; in particular, an α,α- dimethylbenzyl group is preferred.

A phenothiazine-based antioxidant wherein $R^3$ is a hydrogen atom can be produced by the method described in Patent Document 6. For example, α-methylstyrene is allowed to act on phenothiazine in the presence of an acidic catalyst to form 3,7-bis(α,α-dimethylbenzyl)-10H-phenothiazine (hereinafter abbreviated as CD-S), and the sulfur atom is then oxidized with an oxidizing agent, whereby 3,7-bis(α,α-dimethylbenzyl)-10H-phenothiazine-5,5-dioxide (hereinafter abbreviated as CD-$SO_2$) can be produced.

In the case of a phenothiazine-based antioxidant wherein $R^3$ is an aliphatic hydrocarbon group having 1 to 20 carbon atoms, a base is allowed to act on CD-S, and the resultant is then N-alkylated with a halide of an aliphatic hydrocarbon having 1 to 20 carbon atoms, followed by oxidation of the sulfur atom, whereby the desired phenothiazine antioxidant can be produced. Moreover, a base may be allowed to act on CD-$SO_2$ obtained in Patent Document 6, and then a halide of an aliphatic hydrocarbon having 1 to 20 carbon atoms may be allowed to act thereon.

A phenothiazine-based antioxidant wherein $R^3$ is an acyl group represented by the general formula [III] can be produced by the method described in Patent Document 12. Specifically, CD-S is N-acylated with an acyl halide in the presence of a basic organic compound or a basic inorganic compound, followed by oxidization of the sulfur atom, whereby the desired phenothiazine antioxidant can be produced.

It can also be produced by N-acylation from CD-$SO_2$ in a similar manner.

Specific examples of the phenothiazine-based antioxidant represented by the general formula [III] include:

3,7-bis(α,α-dimethylbenzyl)-10H-phenothiazine-5,5-dioxide,
10-methyl-3,7-bis(α,α-dimethylbenzyl)-10H-phenothiazine-5,5-dioxide,
10-n-propyl-3,7-bis(α,α-dimethylbenzyl)-10H-phenothiazine-5,5-dioxide,
10-isopropyl-3,7-bis(α,α-dimethylbenzyl)-10H-phenothiazine-5,5-dioxide,
10-benzyl-3,7-bis(α,α-dimethylbenzyl)-10H-phenothiazine-5,5-dioxide,
10-pivaloyl-3,7-bis(α,α-dimethylbenzyl)-10H-phenothiazine-5,5-dioxide,
10-(2,2-dimethylbutanoyl)-3,7-bis(α,α-dimethylbenzyl)-10H-phenothiazine-5,5-dioxide,
10-(1-adamantanecarbonyl)-3,7-bis(α,α-dimethylbenzyl)-10H-phenothiazine-5,5-dioxide, and the like.

The phenothiazine-based antioxidant as the component (B) is used in an amount of about 0.01 to 5 parts by weight, preferably about 0.1 to 5 parts by weight, based on 100 parts by weight of the acrylic elastomer copolymer as the component (A). If the amount of antioxidant is less than this range, thermal oxidative degradation of the crosslinked acrylic rubber leads to a more significant decrease in mechanical properties. In particular, it is difficult to suppress significant hardening degradation in the later stage of thermal oxidative degradation. In contrast, if the antioxidant is used in an amount greater than the above range, not only softening degradation in the early stage of thermal oxidative degradation may be promoted, but also a further effect of suppressing hardening degradation in the later stage of thermal oxidative degradation cannot be expected, which is not economical.

As the polyvalent amine crosslinking agent as the component (C), an aliphatic polyvalent amine compound, a carbonate of an aliphatic polyvalent amine compound, an aliphatic polyvalent amine compound in which the amino group is protected with an organic group, or an aromatic polyvalent amine compound can be used.

Examples of aliphatic polyvalent amine compound include hexamethylenediamine. Further, examples of

carbonates of aliphatic polyvalent amine compound include hexamethylenediamine carbamate. Examples of aliphatic polyvalent amine in which the amino group is protected with an organic group include N,N'-dicinnamylidene-1,6-hexanediamine or the compounds disclosed in Patent Document 11.

Examples of aromatic polyvalent amine compound include 4,4'-methylenedianiline, m-phenylenediamine, p-phenylenediamine, 4,4'-diaminodiphenylether, 4,4'-bis(4-aminophenoxy)biphenyl, m-xylylenediamine, p-xylylenediamine, 1,3,5-benzenetriamine, 4,4'-(m-phenylenediisopropylidene)dianiline, 4,4'-(p-phenylenediisopropylidene)dianiline, 2,2-bis[4-(4-aminophenoxy)phenyl]propane, 4,4'-diaminobenzanilide, and the like.

The polyvalent amine compounds mentioned above can be used singly or in combination of two or more. Preferably, hexamethylenediamine carbamate, 4,4'-diaminodiphenylether, and 2,2-bis[4-(4-aminophenoxy)phenyl]propane are used.

The amount of the above crosslinking agent added is suitably adjusted depending on the desired crosslinking rate, mechanical strength of the crosslinked product, compression set resistance characteristics and thermal oxidative degradation resistance.

The polyvalent amine crosslinking agent as the component (C) is used at about 0.01 to 5 parts by weight, preferably about 0.05 to 3 parts by weight, based on 100 parts by weight of the acrylic elastomer copolymer as the component (A). If the amount of the polyvalent amine crosslinking agent is less than this range, no improvement is expected in compression set characteristics. In contrast, if the amount thereof used is greater than this range, the thermal oxidative degradation resistance of acrylic rubber may be deteriorated.

Examples of the crosslinking accelerator as the component (D) include crosslinking accelerators, such as guanidine compounds, diazabicycloalkene compounds, or organic acid salts thereof.

Examples of guanidine compound include tetramethylguanidine, tetraethylguanidine, 1,3-diphenylguanidine, 1,3-di-o-tolylguanidine, and the like. Preferably 1,3-diphenylguanidine, 1,3-di-o-tolylguanidine, or a combination thereof.

The diazabicycloalkene compound is preferably 1,8-diazabicyclo[5.4.0]-7-undecene.

The organic acid salt of diazabicycloalkene compound is preferably an organic acid salt of 1,8-diazabicyclo [5.4.0]-7-undecene.

Examples of the organic acid used in the organic acid salt of 1,8-diazabicyclo[5.4.0]-7-undecane include organic monobasic acids or organic dibasic acids.

Examples of the organic monobasic acid include n-hexanoic acid, n-heptanoic acid, n-octanoic acid, 2-ethylhexanoic acid, n-capric acid, n-lauric acid, p-toluenesulfonic acid, phenol, and the like. Examples of the organic dibasic acid include adipic acid, pimelic acid, suberic acid, azelaic acid, sebacic acid, undecanedioic acid, dodecanedioic acid, terephthalic acid, orthophthalic acid, phthalic acid, and the like. Preferable examples are monocarboxylic acids or dicarboxylic acids having 6 to 18 carbon atoms.

The crosslinking accelerator as the component (D) is used in an amount of about 0.1 to 5 parts by weight, preferably about 0.3 to 3 parts by weight, based on 100 parts by weight of the acrylic elastomer copolymer as the component (A). If the amount of the crosslinking accelerator is less than this range, the crosslinking rate may be significantly reduced, the mechanical properties of the acrylic rubber after crosslinking may be reduced, and the mechanical properties after heat aging may be reduced. In contrast, if the crosslinking accelerator is used in an amount greater than the this range, the thermal oxidative degradation resistant or compression set resistance characteristics of the acrylic rubber may be worsened.

The crosslinkable acrylic rubber composition of the present invention may be compounded with, if necessary, various additives, such as fillers, processing aids, plasticizers, softeners, colorants, stabilizers, adhesion aids, mold release agents, conductivity imparting agents, thermal conductivity imparting agents, surface non-adhesives, tackifiers, flexibility imparting agents, heat resistance improving agents, flame retardants, UV absorbers, oil resistance improving agents, scorch retarders, and lubricants.

Examples of the filler include silica, such as basic silica and acidic silica; metal oxides, such as zinc oxide, calcium oxide, titanium oxide, and aluminum oxide; metal hydroxides, such as magnesium hydroxide, aluminum hydroxide, and calcium hydroxide; carbonates, such as magnesium carbonate, aluminum carbonate, calcium carbonate, and barium carbonate; silicates, such as magnesium silicate, calcium silicate, sodium silicate, and aluminum silicate; sulfates, such as aluminum sulfate, calcium sulfate, and barium sulfate; metal sulfides, such as molybdenum disulfide, iron sulfide, and copper sulfide; synthetic hydrotalcite; diatomaceous earth, asbestos, lithopone (zinc sulfide/barium sulfide), graphite, carbon black (MT carbon black, SRF carbon black, FEF carbon black, etc.), fluorinated carbon, calcium fluoride, coke, quartz fine powder, zinc white, talc, mica powder, wollastonite, carbon fiber, aramid fiber, various whiskers, glass fiber, organic reinforcing agents, organic fillers, and the like.

Examples of the processing aid include higher fatty acids, such as stearic acid, oleic acid, palmitic acid, and lauric acid; higher fatty acid salts, such as sodium stearate and zinc stearate; higher fatty acid amides, such as amide stearate and amide oleate; higher fatty acid esters, such as ethyl oleate; higher aliphatic amines, such as stearyl amine and oleyl amine; petroleum-based waxes, such as carnauba wax and ceresin wax; polyglycols, such as ethylene glycol, glycerol, and diethylene glycol; aliphatic hydrocarbons, such as vaseline and paraffin; silicone-based oils, silicone-based polymer,

low-molecular-weight polyethylene, phthalic acid esters, phosphoric acid esters, rosin, (halogenated) dialkyl amine, (halogenated) dialkyl sulfone, surfactants, and the like.

Examples of the plasticizer include epoxy resin, and derivatives of phthalic acid and sebacic acid and the like. Examples of the softener include lubricating oil, process oil, coal tar, castor oil, and calcium stearate and the like. Examples of the antioxidant include phenylenediamines, phosphates, quinolines, cresols, phenols, dithiocarbamate metal salts, and the like.

The above compounding agents used as needed are compounded in the crosslinkable acrylic rubber composition of the present invention comprising acrylic rubber, a phenothiazine-based antioxidant, crosslinking agent, and a crosslinking accelerator, and the resulting mixture is mixed with a Banbury mixer, a pressure kneader, an open roll, or the like. The obtained crosslinkable mixture is crosslinked by primary crosslinking at about 120 to 250°C for about 1 to 60 minutes and optionally oven crosslinking (secondary crosslinking) at about 120 to 200°C for about 1 to 20 hours.

EXAMPLES

Next, the present invention will be described in detail with reference to Examples. The present invention, including its effects, is not limited to the Examples.

Reference Example 1

Production of compound (a)

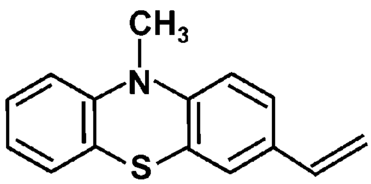

(a)

The compound (a) was produced by the following method.

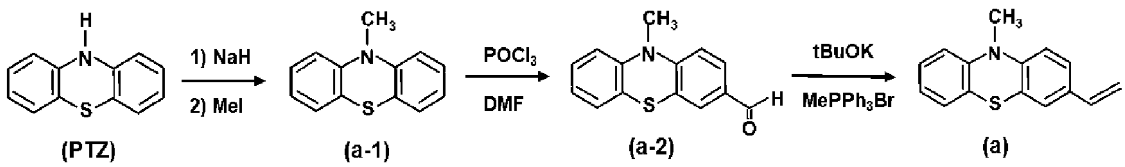


[First step] [PTZ] → (a-1):

In a 1000 ml four-necked flask equipped with a magnetic stirrer, a thermometer, a dropping funnel, and nitrogen gas inlet and outlet tubes, 60.0 g (301 mmol) of phenothiazine [PTZ] and 300 ml of N,N-dimethylformamide were charged, and the temperature in the system under nitrogen atmosphere was cooled to 5°C or less. While keeping the system temperature at 10°C or less, 10.9 g (452 mmol) of sodium hydride was added, followed by reaction for 1 hour. While keeping the system temperature at 20°C or less, 51.3 g (361 mmol) of iodomethane was added dropwise, followed by further reaction for 1 hour. After the reaction was completed, the reaction mixture was added to a saturated aqueous sodium chloride solution. The precipitated colorless solid was filtered off and dissolved in ethyl acetate. After the resultant was washed with a saturated aqueous sodium chloride solution, the organic layer was dried over anhydrous magnesium sulfate, and the insoluble matter was then filtered off. Volatile components were distilled off from the filtrate under reduced pressure to obtain 67.8 g (crude yield: 106%) of a crude product. Recrystallization was performed using ethanol, thereby obtaining 59.9 g (yield: 93%) of 10-methyl-10H-phenothiazine (a-1) as colorless needle crystals.

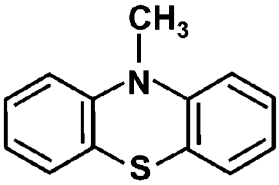

(a-1)

$^1H$ NMR(400MHz, Acetone-d6, δ ppm) :

3.39 (s, 3H, N-C$\underline{H}_3$)
6.91-6.98 (m, 4H, Ar)
7.14 (dd, J=7.6Hz, J=1.6Hz, 2H, Ar)
7.21 (td, J=7.6Hz, J=1.6Hz, 2H, Ar)

[Second step] (a-1) → (a-2):

In a 500 ml four-necked flask equipped with a magnetic stirrer, a dropping funnel, a thermometer, a gas inlet - a gas outlet, and a reflux cooling tube, 210 ml of N,N-dimethylformamide was charged. While keeping the internal temperature in the system at 10°C or less under nitrogen atmosphere, 129.2 g (843 mmol) of phosphoryl chloride was added dropwise, followed by further reaction for 30 minutes. Next, 30 g (141 mmol) of the N-methyl-10H-phenothiazine (a-1) obtained in the first step above was added, followed by reaction at 60°C for 24 hours. After the reaction was completed, the content was poured into an aqueous sodium acetate solution, and sodium hydrogen carbonate was further added for neutralization. The product was extracted from the obtained aqueous solution using ethyl acetate, and the organic layer was washed once with a saturated aqueous sodium chloride solution. The organic layer was dried over anhydrous magnesium sulfate, and the insoluble matter was filtered off, after which volatile components were distilled off from the filtrate under reduced pressure, thereby obtaining 33.8 (crude yield: 99%) of a crude product as a red oily substance. Low-Rf components were removed from the crude product by column chromatography using ethyl acetate as the eluent (carrier: Wakogel C300), thereby obtaining 33.6 g (yield: 98%) of the target low-Rf components removed crude product as a yellow solid. Further, recrystallization was performed using 70 ml of ethyl acetate, thereby obtaining 30.1 g (yield: 88%) of 10-methyl-10H-phenothiazine-3-carbaldehyde (a-2) as yellow crystals.

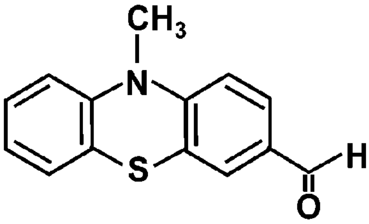

(a-2)

$^1H$ NMR(400MHz, Acetone d6, δ ppm) :

3.49 (s, 3H, N-C$\underline{H}_3$)
7.00-7.06 (m, 2H, Ar)
7.10 (d, J=8.4Hz, 1H, Ar)
7.15-7.19(m, 1H, Ar)
7.22-7.28(m, 1H, Ar)
7.61(d, J=1.6Hz, 1H, Ar)
7.75(dd, J=8.4Hz, J=1.6Hz, 1H, Ar)
9.85(s, 1H, -CHO)

[Third step] (a-2) → (a):

In a 500-ml four-necked flask equipped with a magnetic stirrer, a thermometer, and gas inlet and outlet tubes, 220 ml of tetrahydrofuran was placed, and the internal temperature was cooled to 10°C or less while the inside of the reaction container was replaced with nitrogen. 8.4 g (74.9 mmol) of potassium tert-butoxide and then 26.9 g (75.3 mmol) of methyltriphenylphosphonium bromide were added, followed by reaction for 30 minutes. 15.0 g (62.2 mmol) of the compound (a-2) was added thereto, followed by reaction at 10 to 30°C for 2 hours. The obtained reaction mixture was added to a saturated aqueous sodium chloride solution, and the product was extracted with dichloromethane. After drying

over anhydrous magnesium sulfate, the insoluble matter was filtered off, and volatile components were distilled off from the filtrate under reduced pressure to obtain 35.1 g of residue. The residue was subjected to column chromatography using dichloromethane as the eluent (carrier: Wakogel C300), and triphenylphosphine oxide was removed, followed by recrystallization using ethanol, thereby obtaining 8.7 g (yield: 58%) of a compound (a) as a pale yellow solid.

$^1$H NMR(400MHz, $CDCl_3$, $\delta$ ppm) :

3.37 (s, 3H, N-C$\underline{H}_3$)
5.14 (d, J=10.8Hz, 1H,
C$\underline{H}_2$=CH-Ph (trans against the phenyl group))
5.61 (d, J=17.6Hz, 1H,
C$\underline{H}_2$=CH-Ph (cis against the phenyl group))
6.59 (dd, J=10.8Hz, 17.6Hz, 1H, $CH_2$=C$\underline{H}$-Ph)
6.75 (d, J=8.4Hz, 1H, Ar)
6.81 (d, J=9.2Hz, 1H, Ar)
6.92 (td, J=7.6Hz, 1.2Hz, 1H, Ar)
7.11-7.23 (m, 4H, Ar)

Reference Example 2

Production of 10-pivaloyl-3,7-bis($\alpha$,$\alpha$-dimethylbenzyl)-10H-phenothiazine-5,5-dioxide [CD-$SO_2$-PIV]

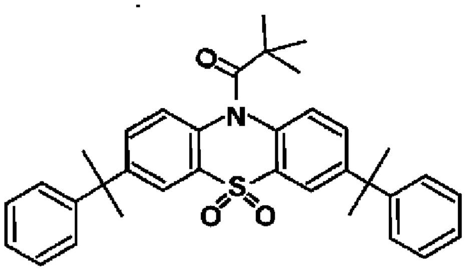

[CD-$SO_2$-PIV]

In a 1000 ml four-necked flask equipped with a magnetic stirrer, a thermometer, nitrogen gas inlet and outlet, and a reflux cooling tube, 119.6 g (0.6 mol) of phenothiazine, 2.88 g of p-toluenesulfonic acid, and 480 ml of toluene were charged and the mixture was heated to 80°C. Then, 141.9 g (1.2 mol) of $\alpha$-methylstyrene was added, and the mixture was reacted in a nitrogen gas atmosphere for 1 hour.

After the reaction mixture was cooled down to the room temperature, the toluene was distilled off under reduced pressure to obtain 265.5 g of a purple solid reaction product. The reaction product was recrystallized with 1500 ml of ethanol, thereby obtaining 179 g (yield: 68 %) of crude 3,7-bis($\alpha$,$\alpha$-dimethylbenzyl)-10H-phenothiazine [CD-S] as pale red purple crystals. Further, the crude CD-S was recrystallized again with ethanol to obtain 161 g of purified CD-S as colorless flaky crystals.

In a 500 ml three-necked flask equipped with a magnetic stirrer, a thermometer, and a reflux cooling tube, 68.7 g (158 mmol) of crude CD-S, 24.8 g (206 mmol) of pivaloyl chloride, and 60 g of pyridine were sequentially charged and reacted at 120°C for 1.5 hours. The pyridine was distilled off from the obtained reaction mixture under reduced pressure, and the residue was then dissolved in 300 ml of dichloromethane. The organic layer was washed three times with 300 ml of a saturated aqueous sodium chloride solution and dried over anhydrous magnesium sulfate. After filtering the magnesium sulfate, volatile components were distilled off from the filtrate under reduced pressure, thereby obtaining 81.7 g of a red highly viscous liquid.

The highly viscous liquid was dissolved in 250 ml of toluene, and the resultant was charged in a 1000 ml three-necked flask equipped with a magnetic stirrer, a thermometer, and a reflux cooling tube. Then, 80 g of acetic acid and 107 g of a 30% hydrogen peroxide solution were sequentially charged, and the mixture was reacted at 100°C for 2 hours.

After the content was cooled down to the room temperature, the upper toluene layer was taken out, and volatile materials were distilled off under reduced pressure. 89 g of the obtained pale red solid was recrystallized with a mixed solvent of ethanol/toluene (volume ratio of 9:1), thereby obtaining 70.3 g (yield on the basis of CD-S: 81%) of CD-$SO_2$-PIV as colorless needle crystals.

$^1$H NMR(300MHz, $CDCl_3$, $\delta$ ppm):

1.22 (s, 9H, $-C(C\underline{H}_3)_3$)
1.71 (s, 12H, $-C(C\underline{H}_3)_2-$)
7.19~7.30 (m, 12H)
7.42 (d, J=9.0Hz, 2H)
8.03 (d, J=1.8Hz, 2H)

Reference Example 3

Production of 3,7-bis(α,α-dimethylbenzyl)-10H-phenothiazine-5,5-dioxide [$CD-SO_2$]

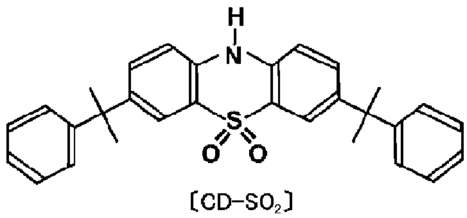

[$CD-SO_2$]

In a 500 ml four-necked flask equipped with a magnetic stirrer, a thermometer, nitrogen gas inlet and outlet, and a reflux cooling tube, 24.9 g (0.125 mol) of phenothiazine, 0.6 g of p-toluenesulfonic acid, and 115 ml of toluene were charged and the mixture was heated to 80°C. Then, 29.5 g (0.25 mol) of α-methylstyrene was added, and the mixture was reacted in a nitrogen gas atmosphere for 1 hour.

Next, 30 g of acetic acid was added to the reaction mixture, then 42.5 g of a 30% hydrogen peroxide solution was added in five batches, and the mixture was further reacted at 80°C for 2 hours. After the content was cooled down to the room temperature and allowed to stand, the upper toluene layer was poured into 500 ml of methanol. After being left at room temperature overnight, 42.5 g (yield: 72%) of crude $CD-SO_2$ was obtained as pale-yellow crystals. This was recrystallized with ethanol to obtain 38 g (yield: 65%) of $CD-SO_2$ as pale-yellow needle-like crystals.

Reference Example 4

[Production of acrylic elastomer copolymer A]

In a separable flask equipped with a thermometer, a stirrer, a nitrogen gas inlet tube, and a Dimroth condenser tube, the following components were charged.

| | | |
|---|---|---|
| Water | | 187 parts by weight |
| Sodium lauryl sulfate | | 2 parts by weight |
| Polyoxyethylene lauryl ether | | 2 parts by weight |
| Charged monomer mixture | | |
| | Ethyl acrylate [EA] | 97.4 parts by weight |
| | Mono n-butyl fumarate [MBF] | 1.6 parts by weight |
| Compound (a) of Reference Example 1 | 1.0 part by weight | |

After oxygen was sufficiently removed from the system by replacement with nitrogen gas, the following components were added.

| | |
|---|---|
| Sodium formaldehyde sulfoxylate (Rongalite, produced by FUJIFILM Wako Pure Chemical Corporation) | 0.008 parts by weight |
| Tertiary butyl hydroperoxide (Perbutyl H69, produced by NOF Corporation) | 0.0047 parts by weight |

Then, a polymerization reaction was initiated at room temperature, and the reaction was continued until the polymerization conversion rate reached 90% or more. The obtained aqueous latex was coagulated with a 10 wt.% sodium sulfate aqueous solution, followed by water washing and drying, thereby obtaining an acrylic rubber A. The Mooney viscosity $PML_{1+4}$ (100°C) of the obtained acrylic elastomer copolymer A was -31.
The molar fraction compositions of compound (a) and EA+MBF were 0.41 mol% and 99.59 mol%, respectively, determined by $^1$H-NMR (400 MHz, Acetone d6, $\delta$ ppm) using the following formulas.

$\alpha$: integral value of signal at 6.5-7.5 ppm
$\beta$: integral value of signal at 3.2-5.0 ppm
Compound (a) (mol%) = 200 x $\alpha$/ ($2\alpha+7\beta$)
EA+MBF (mol%) = 100 - compound (a)

Moreover, the approximate weight fraction compositions of compound (a) and EA+MBF were 1.0 wt% and 99.0 wt%, respectively, determined by the following formulas.

$$\text{Compound (a) (wt\%)} = (\text{compound (a) (mol\%)} \times 239.34 \times 100) / [\text{compound (a) (mol\%)} \times 239.34 + (\text{EA+MBF (mol\%)}) \times 100.8]$$

$$\text{EA+MBF (wt\%)} = 100 - \text{compound (a) (wt\%)}$$

Reference Comparative Example 1

A copolymerization reaction was performed in the same manner as in Reference Example 4, except that the following charged monomer mixture was used, thereby obtaining acrylic rubber B. The Mooney viscosity $PML_{1+4}$ (100°C) of the obtained acrylic elastomer copolymer B was 32.
Charged monomer mixture

| | |
|---|---|
| Ethyl acrylate [EA] | 98.4 parts by weight |
| Mono n-butyl fumarate [MBF] | 1.6 parts by weight |

Reference Example 5

[Production of acrylic elastomer copolymer C]

A copolymerization reaction was performed in the same manner as in Reference Example 4, except that the following charged monomer mixture was used, thereby obtaining acrylic rubber C. The Mooney viscosity $PML_{1+4}$ (100°C) of the obtained acrylic elastomer copolymer C was 30.

| | |
|---|---|
| Ethyl acrylate [EA] | 97.9 parts by weight |
| Mono n-butyl fumarate [MBF] | 1.6 parts by weight |
| Compound (a) of Reference Example 1 | 0.5 parts by weight |

The molar fraction compositions of compound (a) and EA+MBF were 0.20 mol% and 99.80 mol%, respectively, determined by the formulas used in Reference Example 4. Moreover, the approximate weight fraction compositions of compound (a) and EA+MBF were 0.5 wt% and 99.5 wt%, respectively, determined by the formulas used in Reference Example 4.

Reference Example 6

[Production of acrylic elastomer copolymer D]

A copolymerization reaction was performed in the same manner as in Reference Example 4, except that the following charged monomer mixture was used, thereby obtaining acrylic rubber D. The Mooney viscosity $PML_{1+4}$ (100°C)

of the obtained acrylic elastomer copolymer D was 26.

| | |
|---|---|
| Ethyl acrylate [EA] | 57.7 parts by weight |
| n-butyl acrylate [BA] | 40.0 parts by weight |
| Mono n-butyl fumarate [MBF] | 1.6 parts by weight |
| Compound (a) of Reference Example 1 | 0.7 parts by weight |

The molar fraction compositions of compound (a) and EA+ BA+MBF were 0.31 mol% and 99.69 mol%, respectively, determined by the formulas used in Reference Example 4. Moreover, the approximate weight fraction compositions of compound (a) and EA+BA+MBF were 0.7 wt% and 99.3 wt%, respectively, determined by the following formulas.

Compound (a) (wt%) = (compound (a) (mol%) x 239.34 x 100)/ [compound (a) (mol%) x 239.34 + (EA+ BA+MBF (mol %)) x 110.6)]

$$\text{EA+ BA+MBF (wt\%)} = 100 - \text{compound (a) (wt\%)}$$

Reference Comparative Example 2

A copolymerization reaction was performed in the same manner as in Reference Example 4, except that the following charged monomer mixture was used, thereby obtaining acrylic rubber E. The Mooney viscosity $PML_{1+4}$ (100°C) of the obtained acrylic elastomer copolymer E was 26.

Charged monomer mixture

| | |
|---|---|
| Ethyl acrylate [EA] | 58.4 parts by weight |
| n-butyl acrylate [BA] | 40.0 parts by weight |
| Mono n-butyl fumarate [MBF] | 1.6 parts by weight |

Reference Example 7

[Production of acrylic elastomer copolymer F]

A copolymerization reaction was performed in the same manner as in Reference Example 4, except that the following charged monomer mixture was used, thereby obtaining acrylic rubber F. The Mooney viscosity $PML_{1+4}$ (100°C) of the obtained acrylic elastomer copolymer F was 24.

| | |
|---|---|
| Ethyl acrylate [EA] | 57.9 parts by weight |
| n-butyl acrylate [BA] | 40.0 parts by weight |
| Mono n-butyl fumarate [MBF] | 1.6 parts by weight |
| Compound (a) of Reference Example 1 | 0.5 parts by weight |

The molar fraction compositions of compound (a) and EA+BA+MBF were 0.24 mol% and 99.76 mol%, respectively, determined by the formulas used in Reference Example 4. Moreover, the approximate weight fraction compositions of compound (a) and EA+BA+MBF were 0.5 wt% and 99.5 wt%, respectively, determined by the formulas used in Reference Example 6.

Example 1

| | |
|---|---|
| Acrylic elastomer copolymer A | 100 parts by weight |

(continued)

| | |
|---|---|
| SRF carbon black (Seast GS, produced by Tokai Carbon Co., Ltd.) | 60 parts by weight |
| Stearic acid (TST, produced by Miyoshi Oil & Fat Co., Ltd.) | 1 part by weight |
| Polyoxyethylene stearyl ether phosphate (Phosphanol RL-210, produced by Toho Chemical Industry Co., Ltd.) | 0.5 parts by weight |
| Crosslinking accelerator (Vulcofac ACT55, produced by Safic-Alcan) | 1 part by weight |
| Hexamethylenediamine carbamate (Cheminox AC6F, produced by Unimatec Co., Ltd.) | 0.6 parts by weight |
| CD-$SO_2$-PIV | 1 part by weight |

Among the above components, the acrylic elastomer copolymer A, SRF carbon black, stearic acid, and polyoxyethylene stearyl ether phosphate were mixed with a Banbury mixer. The obtained mixture and the other components were mixed using an open roll, thereby obtaining a crosslinkable acrylic rubber composition.

The obtained composition was subjected to primary crosslinking at 180°C for 8 minutes using a 100-ton press molding machine. Further, oven crosslinking was carried out at 175°C for 4 hours to obtain a sheet-like crosslinked product having a thickness of about 2 mm, and a cylindrical crosslinked product having a diameter of about 29 mm and a height of about 12.5 mm.

The crosslinking characteristics of the acrylic rubber composition and the physical properties of its crosslinked product were measured in the following manner.

Mooney scorch test: according to JIS K6300-1 corresponding to ISO 289-1 (125°C). Using a Mooney viscometer (AM-3, produced by Toyo Seiki Seisaku-sho, Ltd.), the minimum Mooney viscosity (ML min) and scorch time (t5) values were measured.

Crosslinking test: according to JIS K6300-2 corresponding to ISO 6502 (180°C, 12 minutes). Using a rotorless rheometer (RLR-3, produced by Toyo Seiki Seisaku-sho, Ltd.), ML, MH, tc (10), and tc (90) values were measured.

ML: minimum torque
MH: maximum torque
tc (10): time required for the crosslinking torque to reach ML + (MH - ML) x 0.1
tc (90): time required for the crosslinking torque to reach ML + (MH - ML) x 0.9

Normal state physical properties: measured for the post cured sheet according to

JIS K6251 corresponding to ISO 37and JIS K6253 corresponding to ISO 37

Air heating aging test: measured for the post cured sheet according to JIS K6257 corresponding to ISO 188 (190°C: 100 hours, 200 hours, 300 hours, 400 hours, 500 hours, 600 hours)

Oil dipping test: after an oil (IRM 903 oil) dipping was performed at 150°C for 168 hours according to JIS K6258 corresponding to ISO 1817, each of change rates and the volumetric swelling rate from normal state physical properties before oil dipping were determined.

Air heating aging test - oil dipping - air heating aging combined test:
after an air heating aging test was performed at 175°C for 150 hours according to JIS K6257 corresponding to ISO 188, an oil (IRM 903 oil) dipping test was performed at 150°C for 168 hours according to JIS K6258 corresponding to ISO 1817, an air heating aging test was further preformed at 190°C for 300 hours according to JIS K6257 corresponding to ISO 188, and each of change rates from the normal state physical properties were determined.

Compression set test: according to JIS K6262 corresponding to ISO 815-1 (175°C: 70 hours, 500 hours)

Comparative Example 1

In Example 1, the CD-$SO_2$-PIV was not used.

Comparative Example 2

In Example 1, the acrylic elastomer copolymer B was used in place of the acrylic elastomer copolymer A.

Comparative Example 3

In Comparative Example 2, 1 part by weight of 4,4'-bis(α,α-dimethylbenzyl)diphenylamine (Nocrac CD, produced by Ouchi Shinko Chemical Industrial Co., Ltd.) was used in place of the $CD-SO_2$-PIV.

Example 2

In Example 1, each of the following components were used for the crosslinkable acrylic rubber composition.

| | |
|---|---|
| Acrylic elastomer copolymer C | 100 parts by weight |
| SRF carbon black (Seast GS) | 70 parts by weight |
| Stearic acid (TST) | 1 part by weight |
| Polyoxyethylene stearyl ether phosphate (Phosphanol RL-210) | 0.5 parts by weight |
| Crosslinking accelerator (Vulcofac ACT55) | 1 part by weight |
| 2,2-bis[4-(4-aminophenoxy)phenyl]propane (produced by Tokyo Chemical Industry Co., Ltd.) | 1.2 parts by weight |
| $CD-SO_2$ | 1 part by weight |

Comparative Example 4

In Example 2, the $CD-SO_2$ was not used.

Comparative Example 5

In Example 2, the acrylic elastomer copolymer B was used in place of the acrylic elastomer copolymer C.

Comparative Example 6

In Comparative Example 5, 1 part by weight of 4,4'-bis(α,α-dimethylbenzyl)diphenylamine (Nocrac CD) was used in place of the $CD-SO_2$.

Example 3

In Example 1, each of the following components were used for the crosslinkable acrylic rubber composition.

| | |
|---|---|
| Acrylic elastomer copolymer D | 100 parts by weight |
| SRF carbon black (Seast GS) | 70 parts by weight |
| Stearic acid (TST) | 1 part by weight |
| Polyoxyethylene stearyl ether phosphate (Phosphanol RL-210) | 0.5 parts by weight |
| Stearyl amine (Farmin 80S) | 1 part by weight |
| Crosslinking accelerator (Vulcofac ACT55) | 1 part by weight |
| Hexamethylenediamine carbamate (Cheminox AC6F) | 0.6 parts by weight |
| $CD-SO_2$ | 1 part by weight |

Comparative Example 7

In Example 3, the $CD-SO_2$ was not used.

Comparative Example 8

In Example 3, the acrylic elastomer copolymer E was used in place of the acrylic elastomer copolymer D.

Comparative Example 9

In Comparative Example 8, 1 part by weight of 4,4'-bis(α,α-dimethylbenzyl)diphenylamine (Nocrac CD) was used in place of the $CD-SO_2$.

Example 4

In Example 1, each of the following components were used for the crosslinkable acrylic rubber composition, and further a toluene dipping-air heating aging combined test was performed on the crosslinked product of the acrylic rubber composition.

| | |
|---|---|
| Acrylic elastomer copolymer F | 100 parts by weight |
| SRF carbon black (Seast GS) | 70 parts by weight |
| Stearic acid (TST) | 1 part by weight |
| Polyoxyethylene stearyl ether phosphate (Phosphanol RL-210) | 0.5 parts by weight |
| Crosslinking accelerator (Vulcofac ACT55) | 1 part by weight |
| 2,2-bis[4-(4-aminophenoxy)phenyl]propane (produced by Tokyo Chemical Industry Co., Ltd.) | 1.2 parts by weight |
| CD-$SO_2$ | 1 part by weight |

Toluene dipping-air heating aging combined test: after a toluene dipping test was performed at room temperature for 168 hours according to JIS K6258 corresponding to ISO 1817, toluene was removed from the test piece by air drying, and an air heating aging test was further preformed at 190°C for 300 hours according to JIS K6257 corresponding to ISO 188.

Comparative Example 10

In Example 4, the CD-$SO_2$ was not used.

Comparative Example 11

In Example 4, the acrylic elastomer copolymer E was used in place of the acrylic elastomer copolymer F.

Comparative Example 12

In Comparative Example 11, 1 part by weight of 4,4'-bis($\alpha$,$\alpha$-dimethylbenzyl)diphenylamine (Nocrac CD) was used in place of the CD-$SO_2$.

Following Tables 1 to 4 and Figs 1 to 15 show the results obtained in the above Examples 1 to 4 and Comparative Examples 1 to 12.

Table 1

| Measurement results | | Example | Comp arative Exa mple | | |
|---|---|---|---|---|---|
| | | 1 | 1 | 2 | 3 |
| Mooney scorch test (125°C) | | | | | |
| ML min | (pts) | 65 | 65 | 61 | 64 |
| t5 | (min) | 1.6 | 1.5 | 2.8 | 2.4 |
| Crosslinking test (180°C) | | | | | |
| tc (10) | (min) | 0.53 | 0.52 | 0.50 | 0.50 |
| tc (90) | (min) | 5.34 | 5.43 | 4.76 | 4.87 |
| ML | (N·m) | 0.23 | 0.24 | 0.21 | 0.23 |
| MH | (N·m) | 0.94 | 0.96 | 0.94 | 0.97 |
| Normal state physical properties (post cured) | | | | | |
| Hardness | (Duro A) | 65 | 65 | 62 | 63 |
| 100% modulus | (MPa) | 4.9 | 5.2 | 4.3 | 4.4 |
| Strength at break | (MPa) | 17.0 | 17.1 | 16.1 | 15.8 |
| Elongation at break | (%) | 260 | 260 | 240 | 260 |
| Air heating aging test (190°C, 100 hours) | | | | | |
| Hardness change | (Duro A) | +12 | +9 | +4 | +0 |
| Rate of change in 100% modulus | (%) | +65 | +62 | -19 | -36 |

(continued)

| | | | | | |
|---|---|---|---|---|---|
| Air heating aging test (190°C, 100 hours) | | | | | |
| Rate of change in strength at break | (%) | -12 | -8 | -27 | -35 |
| Rate of change in elongation at break | (%) | -33 | -30 | +25 | +28 |
| Air heating aging test (190°C, 200 hours) | | | | | |
| Hardness change | (Duro A) | +11 | +10 | +5 | +0 |
| Rate of change in 100% modulus | (%) | +51 | +37 | -37 | -52 |
| Rate of change in strength at break | (%) | -21 | -23 | -43 | -60 |
| Rate of change in elongation at break | (%) | -28 | -29 | +46 | +31 |
| Air heating aging test (190°C, 300 hours) | | | | | |
| Hardness change | (Duro A) | +13 | +13 | +6 | +3 |
| Rate of change in 100% modulus | (%) | +29 | +17 | -51 | -48 |
| Rate of change in strength at break | (%) | -34 | -41 | -60 | -70 |
| Rate of change in elongation at break | (%) | -25 | -29 | +52 | +33 |
| Air heating aging test (190°C, 400 hours) | | | | | |
| Hardness change | (Duro A) | +15 | +16 | +9 | +15 |
| Rate of change in 100% modulus | (%) | +16 | +8 | -51 | -25 |
| Rate of change in strength at break | (%) | -45 | -59 | -70 | -69 |
| Rate of change in elongation at break | (%) | -23 | -42 | +56 | -11 |
| Air heating aging test (190°C, 500 hours) | | | | | |
| Hardness change | (Duro A) | +18 | +24 | +9 | +23 |
| Rate of change in 100% modulus | (%) | +12 | +31 | -56 | +14 |
| Rate of change in strength at break | (%) | -53 | -58 | -76 | -65 |
| Rate of change in elongation at break | (%) | -29 | -61 | +64 | -51 |
| Air heating aging test (190°C, 600 hours) | | | | | |
| Hardness change | (Duro A) | +19 | +23 | +17 | +22 |
| Rate of change in 100% modulus | (%) | +4 | | -47 | |
| Rate of change in strength at break | (%) | -60 | -54 | -80 | -61 |
| Rate of change in elongation at break | (%) | -34 | -72 | +35 | -77 |
| Oil dipping test (150°C, 168 hours) | | | | | |
| Hardness change | (Duro A) | -6 | -6 | -6 | -7 |
| Rate of change in 100% modulus | (%) | -2 | -6 | -5 | +5 |
| Rate of change in strength at break | (%) | -9 | -10 | -11 | -6 |
| Rate of change in elongation at | break (%) | -3 | -4 | +1 | -6 |
| Volumetric swelling rate | (%) | +11 | +11 | +11 | +10 |
| Air heating aging test-oil dipping-air heating aging combined test | | | | | |
| Hardness change | (Duro A) | +17 | +17 | +12 | +19 |
| Rate of change in 100% modulus | (%) | +24 | +15 | -40 | +9 |
| Rate of change in strength at break | (%) | -51 | -51 | -65 | -68 |
| Rate of change in elongation at | break (%) | -37 | -39 | +27 | -60 |
| Compression set test | | | | | |
| 175°C, 70 hours | (%) | 17 | 16 | 14 | 15 |
| 175°C, 500 hours | (%) | 34 | 34 | 27 | 27 |

Table 2

| Measurement results | | Example | Comparative Example | | |
|---|---|---|---|---|---|
| | | 2 | 4 | 5 | 6 |
| Mooney scorch test (125°C) | | | | | |
| ML min | (pts) | 62 | 62 | 63 | 63 |

(continued)

| Measurement results | | Example 2 | Comparative Example 4 | Comparative Example 5 | Comparative Example 6 |
|---|---|---|---|---|---|
| Mooney scorch test (125°C) | | | | | |
| t5 | (min) | 6.4 | 5.7 | 6.3 | 5.0 |
| Crosslinking test (180°C) | | | | | |
| tc (10) | (min) | 1.08 | 1.03 | 1.25 | 1.16 |
| tc (90) | (min) | 8.21 | 8.11 | 8.26 | 8.13 |
| ML | (N·m) | 0.27 | 0.28 | 0.26 | 0.27 |
| MH | (N·m) | 0.82 | 0.90 | 0.88 | 0.93 |
| Normal state physical properties (post cured) | | | | | |
| Hardness | (Duro A) | 69 | 70 | 66 | 66 |
| 100% modulus | (MPa) | 6.0 | 6.3 | 6.7 | 5.2 |
| Strength at break | (MPa) | 16.8 | 17.4 | 16.8 | 16.2 |
| Elongation at break | (%) | 220 | 230 | 220 | 230 |
| Air heating aging test (190°C, 100 hours) | | | | | |
| Hardness change | (Duro A) | +12 | +11 | +10 | +5 |
| Rate of change in 100% modulus | (%) | +15 | +22 | -33 | -25 |
| Rate of change in strength at break | (%) | -16 | -11 | -29 | -28 |
| Rate of change in elongation at break | (%) | -10 | -14 | +27 | +20 |
| Air heating aging test (190°C, 200 hours) | | | | | |
| Hardness change | (Duro A) | +12 | +13 | +9 | +9 |
| Rate of change in 100% modulus | (%) | +10 | +0 | -42 | -42 |
| Rate of change in strength at break | (%) | -26 | -29 | -40 | -56 |
| Rate of change in elongation at break | (%) | -13 | -12 | +27 | +28 |
| Air heating aging test (190°C, 300 hours) | | | | | |
| Hardness change | (Duro A) | +12 | +14 | +10 | +13 |
| Rate of change in 100% modulus | (%) | -10 | -25 | -55 | -40 |
| Rate of change in strength at break | (%) | -43 | -59 | -58 | -69 |
| Rate of change in elongation at break | (%) | -8 | -10 | +40 | +11 |
| Air heating aging test (190°C, 400 hours) | | | | | |
| Hardness chang | (Duro A) | +13 | +17 | +13 | +20 |
| Rate of change in 100% modulus | (%) | -25 | -14 | -58 | -12 |
| Rate of change in strength at break | (%) | -56 | -63 | -69 | -67 |
| Rate of change in elongation at break | (%) | +2 | -35 | +50 | -32 |
| Air heating aging test (190°C, 500 hours) | | | | | |
| Hardness change | (Duro A) | +16 | +20 | +13 | +24 |
| Rate of change in 100% modulus | (%) | -23 | | -57 | |
| Rate of change in strength at break | (%) | -63 | -60 | -75 | -60 |
| Rate of change in elongation at break | (%) | -9 | -69 | +32 | -69 |
| Air heating aging test (190°C, 600 hours) | | | | | |
| Hardness change | (Duro A) | +17 | +22 | +17 | +27 |
| Rate of change in 100% modulus | (%) | -25 | | -52 | |
| Rate of change in strength at break | (%) | -69 | -47 | -77 | -43 |
| Rate of change in elongation at break | (%) | -16 | -81 | +14 | -85 |
| Oil dipping test (150°C, 168 hours) | | | | | |
| Hardness change | (Duro A) | -3 | -5 | -3 | -4 |
| Rate of change in 100% modulus | (%) | -5 | -8 | -15 | +12 |
| Rate of change in strength at break | (%) | -7 | -5 | -7 | -1 |

(continued)

| | | | | | |
|---|---|---|---|---|---|
| Oil dipping test (150°C, 168 hours) | | | | | |
| Rate of change in elongation at break | (%) | +4 | +7 | +7 | -3 |
| Volumetric swelling rate | (%) | +11 | +12 | +11 | +11 |
| Compression set test | | | | | |
| 175°C, 70 hours | (%) | 24 | 24 | 22 | 22 |
| 175°C, 500 hours | (%) | 39 | 38 | 34 | 33 |

Table 3

| Measurement results | | Example | Comparative Example | | |
|---|---|---|---|---|---|
| | | 3 | 7 | 8 | 9 |
| Mooney scorch test (125°C) | | | | | |
| ML min | (pts) | 46 | 48 | 47 | 47 |
| t5 | (min) | 4.9 | 4.0 | 4.2 | 3.6 |
| Crosslinking test (180°C) | | | | | |
| tc (10) | (min) | 0.55 | 0.51 | 0.55 | 0.52 |
| tc (90) | (min) | 6.54 | 6.27 | 6.53 | 6.35 |
| ML | (N·m) | 0.231 | 0.22 | 0.21 | 0.21 |
| MH | (N·m) | 0.80 | 0.84 | 0.83 | 0.84 |
| Normal state physical properties (post cured) | | | | | |
| Hardness | (Duro A) | 66 | 65 | 63 | 62 |
| 100% modulus | (MPa) | 4.9 | 5.1 | 4.9 | 4.9 |
| Strength at break | (MPa) | 13.4 | 14.5 | 12.3 | 12.7 |
| Elongation at break | (%) | 230 | 240 | 200 | 220 |
| Air heating aging test (190°C, 100 hours) | | | | | |
| Hardness change | (Duro A) | +8 | +12 | +7 | +6 |
| Rate of change in 100% modulus | (%) | +38 | +29 | -20 | -24 |
| Rate of change in strength at break | (%) | -13 | -13 | -16 | -17 |
| Rate of change in elongation at break | (%) | -29 | -28 | +16 | +14 |
| Air heating aging test (190°C, 200 hours) | | | | | |
| Hardness change | (Duro A) | +9 | +12 | +5 | +5 |
| Rate of change in 100% modulus | (%) | +56 | +57 | -27 | -29 |
| Rate of change in strength at break | (%) | -10 | -14 | -24 | -28 |
| Rate of change in elongation at break | (%) | -29 | -35 | +9 | +6 |
| Air heating aging test (190°C, 300 hours) | | | | | |
| Hardness change | (Duro A) | +10 | +14 | +7 | +9 |
| Rate of change in 100% modulus | (%) | +33 | +39 | -37 | -22 |
| Rate of change in strength at break | (%) | -23 | -30 | -37 | -40 |
| Rate of change in elongation at | break (%) | -28 | -37 | +11 | -14 |
| Air heating aging test (190°C, 400 hours) | | | | | |
| Hardness change | (Duro A) | +12 | +16 | +10 | +16 |
| Rate of change in 100% modulus | (%) | +19 | +35 | -39 | +18 |
| Rate of change in strength at break | (%) | -34 | -39 | -46 | -39 |
| Rate of change in elongation at | break (%) | -27 | -42 | +25 | -36 |
| Air heating aging test (190°C, 500 hours) | | | | | |
| Hardness change | (Duro A) | +11 | +17 | +9 | +18 |
| Rate of change in 100% modulus | (%) | +17 | +53 | -35 | +67 |
| Rate of change in strength at break | (%) | -40 | -43 | -51 | -35 |
| Rate of change in elongation at break | (%) | -31 | -54 | +3 | -54 |

(continued)

| | | | | | |
|---|---|---|---|---|---|
| Air heating aging test (190°C, 600 hours) | | | | | |
| Hardness change | (Duro A) | +13 | +19 | +12 | +23 |
| Rate of change in 100% modulus | (%) | +6 | | -33 | |
| Rate of change in strength at break | (%) | -50 | -46 | -55 | -43 |
| Rate of change in elongation at break | (%) | -36 | -67 | -5 | -73 |
| Oil dipping test (150°C, 168 hours) | | | | | |
| Hardness change | (Duro A) | -18 | -16 | -15 | -15 |
| Rate of change in 100% modulus | (%) | -20 | -21 | -18 | -14 |
| Rate of change in strength at break | (%) | -22 | -22 | -19 | -17 |
| Rate of change in elongation at break | (%) | -14 | -13 | -5 | -14 |
| Volumetric swelling rate | (%) | +28 | +28 | +28 | +27 |
| Compression set test | | | | | |
| 175°C, 70 hours | (%) | 23 | 22 | 21 | 21 |
| 175°C, 500 hours | (%) | 40 | 40 | 37 | 34 |

Table 4

| Measurement results | | Example | Comparative Example | | |
|---|---|---|---|---|---|
| | | 4 | 10 | 11 | 12 |
| Mooney scorch test (125°C) | | | | | |
| ML min | (pts) | 47 | 48 | 50 | 50 |
| t5 | (min) | 7.1 | 7.2 | 7.7 | 8.0 |
| Crosslinking test (180°C) | | | | | |
| tc (10) | (min) | 0.94 | 0.84 | 1.15 | 1.16 |
| tc (90) | (min) | 8.13 | 8.04 | 8.34 | 8.25 |
| ML | (N·m) | 0.21 | 0.21 | 0.20 | 0.19 |
| MH | (N·m) | 0.70 | 0.74 | 0.69 | 0.68 |
| Normal state physical properties (post cured) | | | | | |
| Hardnes | (Duro A) | 66 | 65 | 66 | 65 |
| 100% modulus | (MPa) | 5.0 | 5.7 | 5.8 | 5.4 |
| Strength at break | (MPa) | 13.7 | 14.6 | 13.0 | 13.4 |
| Elongation at break | (%) | 200 | 220 | 190 | 210 |
| Air heating aging test (190°C, 100 hours) | | | | | |
| Hardness change (Duro | A) | +7 | +11 | +4 | +6 |
| Rate of change in 100% modulus | (%) | +26 | +18 | -31 | -33 |
| Rate of change in strength at break | (%) | -15 | -6 | -22 | -25 |
| Rate of change in elongation at break | (%) | -15 | -14 | +17 | +20 |
| Air heating aging test (190°C, 200 hours) | | | | | |
| Hardness change (Duro | A) | +8 | +12 | -2 | +8 |
| Rate of change in 100% modulus | (%) | +16 | +12 | -38 | -26 |
| Rate of change in strength at break | (%) | -25 | -25 | -32 | -42 |
| Rate of change in elongation at break | (%) | -11 | -25 | +18 | -1 |
| Air heating aging test (190°C, 300 hours) | | | | | |
| Hardness change (Duro | A) | +10 | +16 | +10 | +17 |
| Rate of change in 100% modulus | (%) | -4 | +23 | -41 | +28 |
| Rate of change in strength at break | (%) | -39 | -42 | -45 | -40 |
| Rate of change in elongation at break | (%) | -9 | -40 | +15 | -41 |
| Air heating aging test (190°C, 400 hours) | | | | | |
| Hardness change (Duro | A) | +15 | +19 | +13 | +22 |
| Rate of change in 100% modulus | (%) | -8 | | -36 | |
| Rate of change in strength at break | (%) | -47 | -44 | -53 | -35 |

(continued)

| Measurement results | | Example 4 | Comparative Example 10 | Comparative Example 11 | Comparative Example 12 |
|---|---|---|---|---|---|
| Rate of change in elongation at break | (%) | -15 | -60 | +2 | -68 |
| Air heating aging test (190°C, 500 hours) | | | | | |
| Hardness change (Duro | A) | +18 | +23 | +16 | +27 |
| Rate of change in 100% modulus | (%) | +2 | | -24 | |
| Rate of change in strength at break | (%) | -50 | -35 | -56 | -26 |
| Rate of change in elongation at break | (%) | -24 | -74 | -19 | -80 |
| Air heating aging test (190°C, 600 hours) | | | | | |
| Hardness change (Duro | A) | +19 | +26 | +17 | +29 |
| Rate of change in 100% modulus | (%) | +4 | | -14 | |
| Rate of change in strength at break | (%) | -55 | -30 | -57 | -8 |
| Rate of change in elongation at break | (%) | -33 | -80 | -34 | -86 |
| Toluene dipping (room temperature, 168 hours) -air heating aging (190°C, 300 hours) combined test | | | | | |
| Hardness change | (Duro A) | +12 | +14 | +12 | +24 |
| Rate of change in 100% modulus | (%) | +18 | +32 | -2 | |
| Rate of change in strength at break | (%) | -35 | -40 | -33 | -37 |
| Rate of change in elongation at break | (%) | -16 | -47 | -13 | -29 |
| Compression set test | | | | | |
| 175°C, 70 hours | (%) | 26 | 25 | 25 | 26 |
| 175°C, 500 hours | (%) | 41 | 41 | 40 | 39 |

Regarding Example 1 and Comparative Examples 1 to 3, ΔΔEb, which indicates the degree of extraction of the antioxidant from the crosslinked product by the oil dipping test, was calculated from the following formula. The results were -12 in Example 1, -10 in Comparative Example 1, -25 in Comparative Example 2, and -95 in Comparative Example 3.

ΔΔEb = ΔEb (rate of change in elongation at break after air heating aging test-oil dipping test-air heating aging combined test) - ΔEb (rate of change in elongation at break after air heating aging test at 190°C for 300 hours)

Further, regarding Example 4 and Comparative Examples 10 to 12, ΔΔEb, which indicates the degree of extraction of the antioxidant from the crosslinked product by the toluene dipping test, was calculated from the following formula. The results were -7 in Example 4, -7 in Comparative Example 10, -28 in Comparative Example 11, and -38 in Comparative Example 12.

ΔΔEb = ΔEb (rate of change in elongation at break after toluene dipping test-air heating aging combined test) - ΔEb (rate of change in elongation at break after air heating aging test at 190°C for 300 hours)

The above results reveal the following.

(1) In the air heating aging test, it can be seen that the decrease in strength at break is suppressed in Example 1 compared to Comparative Examples 2 and 3. This is considered to be mainly due to the crosslinking effect of the antioxidant component chemically bonded to the acrylic elastomer. The same tendency is also found in the comparison between Comparative Examples 5 and 6 and Example 2, Comparative Examples 8 and 9 and Example 3, and Comparative Examples 11 and 12 and Example 4.
(2) In Examples 1 to 3 (after a slight decrease in elongation at break in the early stage of the test), the value remains almost constant and the rubber elasticity is maintained for a long period of time, whereas in Comparative Examples 1, 4, and 7, the elongation at break decreases from the middle of the test, indicating that rubber elasticity is lost. On the other hand, in Comparative Examples 2, 3, 5, 6, 8, 9, 11, and 12, a significant increase in elongation at break and a significant decrease in strength at break due to softening degradation were observed from the early stage of the test, suggesting a decrease in the mechanical strength of the crosslinked product.
(3) In the oil dipping test-air heating aging combined test, the significant decrease in elongation at break in

Comparative Example 3 compared to Example 1 and Comparative Example 1 is presumed to be due to the extraction of the antioxidant from the crosslinked product by the oil dipping test. In Comparative Example 2, on the contrary, an increase in elongation at break is observed. This is considered to be because the phenothiazine-based antioxidant is difficult to extract with oil and remains in the rubber. This is also shown by the small absolute value of ΔΔEb, which indicates the degree of extraction of the antioxidant from the crosslinked product by the oil dipping test. On the other hand, in Example 1 and Comparative Example 1, the antioxidant component is chemically bonded to the acrylic elastomer copolymer and thus escapes extraction with oil, and as a result, it is presumed that the decrease in elongation at break is suppressed.

(4) It is considered that the larger the absolute numerical value of ΔΔEb, which indicates the degree of extraction of the antioxidant from the crosslinked product by the toluene dipping test, the more antioxidant is extracted and the residual rate in the crosslinked product is lower. Therefore, these results suggest that the antioxidant component resulting from the copolymerizable aging agent (a) is difficult to extract with toluene, and that most of it remains in the crosslinked product (Example 4 and Comparative Example 10). Further, when comparing Example 4 and Comparative Example 12, the apparent small decrease in elongation at break in Example 4 can be attributed to the effect of the combined use of the copolymerizable aging agent (a) and the phenothiazine-based antioxidant CD-$SO_2$.

(5) The reason why the decrease in strength at break of Example 1 and Comparative Example 1 is smaller than that of Comparative Examples 2 and 3 is considered to be mainly due to the crosslinking effect of the antioxidant component chemically bonded to the acrylic elastomer copolymer. In Comparative Example 2, the decrease in strength at break due to softening degradation is remarkable. This phenomenon is specific to rubber compounded with a phenothiazine-based antioxidant.

(6) The rubber member obtained by crosslinking the composition of the present invention is capable of retaining its mechanical strength and rubber elasticity for a long period of time even under severe thermal oxidative degradation conditions or severe extraction • chemical degradation conditions with liquid media such as oil.

## Claims

1. A crosslinkable acrylic rubber composition comprising:

(A) An acrylic elastomer copolymer comprising a copolymerizable antioxidant represented by general formula [I]:

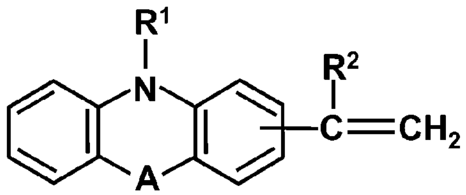

〔I〕

(wherein $R^1$ is a monovalent aliphatic hydrocarbon group having 1 to 20 carbon atoms, $R^2$ is a hydrogen atom or a methyl group, and A is a direct bond, an oxygen atom or a sulfur atom), an alkyl (meth)acrylate monomer and/or an alkoxyalkyl (meth)acrylate monomer, and an α,β-unsaturated carboxylic acid monomer;

(B) a phenothiazine-based antioxidant represented by the general formula [II]:

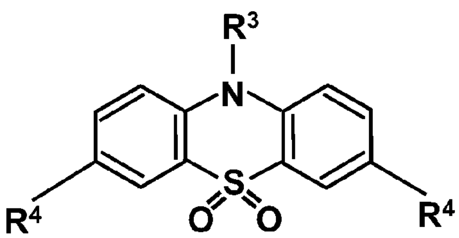

〔II〕

[wherein $R^3$ is a hydrogen atom, a monovalent aliphatic hydrocarbon group having 1 to 20 carbon atoms, an aralkyl group having 7 to 20 carbon atoms, or an acyl group represented by the following general formula [III]:

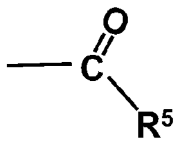

[III]

(wherein $R^5$ is a monovalent aliphatic hydrocarbon group having 1 to 20 carbon atoms), and $R^4$ is an aralkyl group having 7 to 20 carbon atoms];
(C) a polyvalent amine crosslinking agent; and
(D) a crosslinking accelerator.

**2.** The crosslinkable acrylic rubber composition according to claim 1, wherein in the copolymerizable antioxidant represented by the general formula [I], A is a sulfur atom.

**3.** The crosslinkable acrylic rubber composition according to claim 1, wherein the component (A) is an acrylic elastomer copolymer composed of a copolymerizable antioxidant represented by the general formula [I], an alkyl acrylate monomer, and an $\alpha,\beta$-unsaturated carboxylic acid monomer.

**4.** The crosslinkable acrylic rubber composition according to claim 3, wherein an ethyl acrylate and/or n-butyl (meth) acrylate is used as the alkyl acrylate monomer.

**5.** The crosslinkable acrylic rubber composition according to claim 1, wherein the polyvalent amine crosslinking agent as the component (C) is a hexamethylenediamine carbamate, 4,4'-diaminodiphenylether or 2,2-bis[4-(4-aminophenoxy)phenyl]propane.

**6.** The crosslinkable acrylic rubber composition according to claim 1, wherein the crosslinking accelerator as the component (D) is 1,8-diazabicyclo[5.4.0]-7-undecane or an organic acid salt thereof.

[Fig. 1]

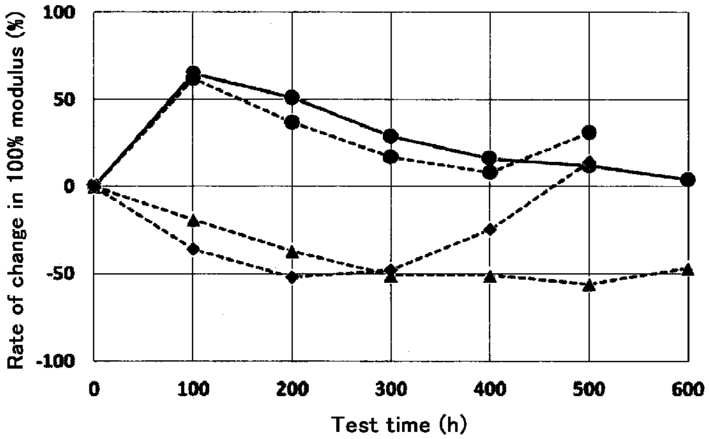
Rate of change in 100% modulus (%)
100
50
0
-50
-100
0
100
200
300
400
500
600
Test time (h)

[Fig. 2]

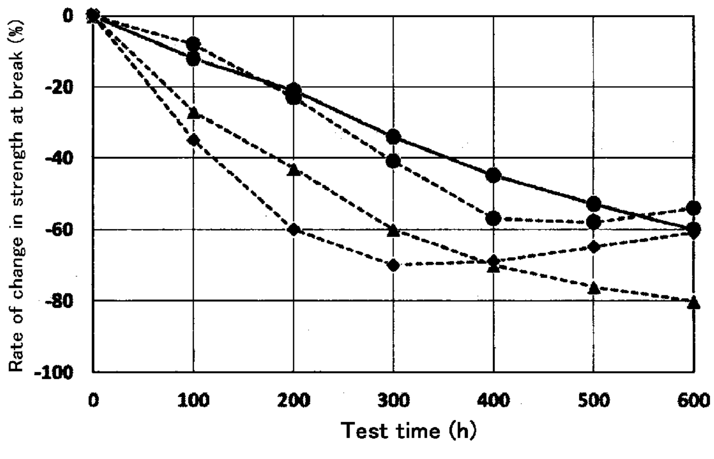
Rate of change in strength at break (%)
0
-20
-40
-60
-80
-100
0
100
200
300
400
500
600
Test time (h)

[Fig. 3]

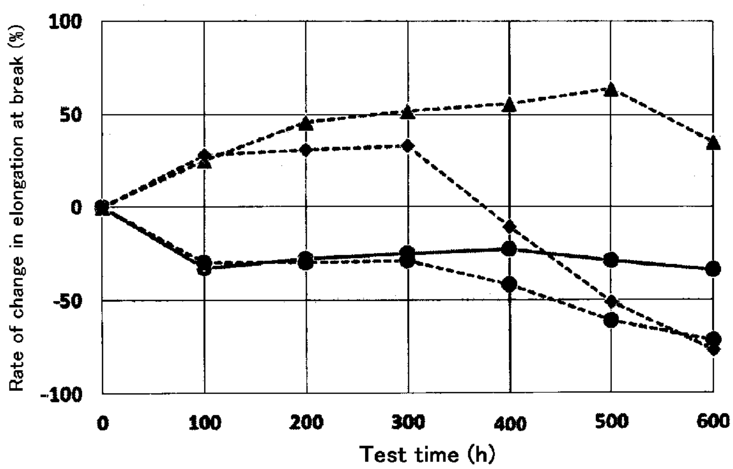
100
50
0
-50
-100
Rate of change in elongation at break (%)
0
100
200
300
400
500
600
Test time (h)

[Fig. 4]

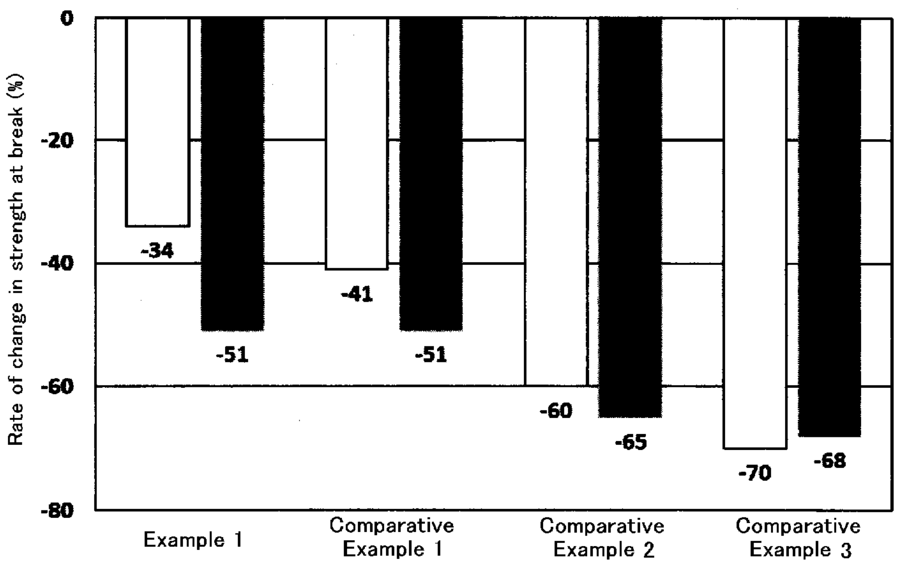
0
-20
-40
-60
-80
Rate of change in strength at break (%)
-34
-51
-41
-51
-60
-65
-70
-68
Example 1
Comparative Example 1
Comparative Example 2
Comparative Example 3

[Fig. 5]

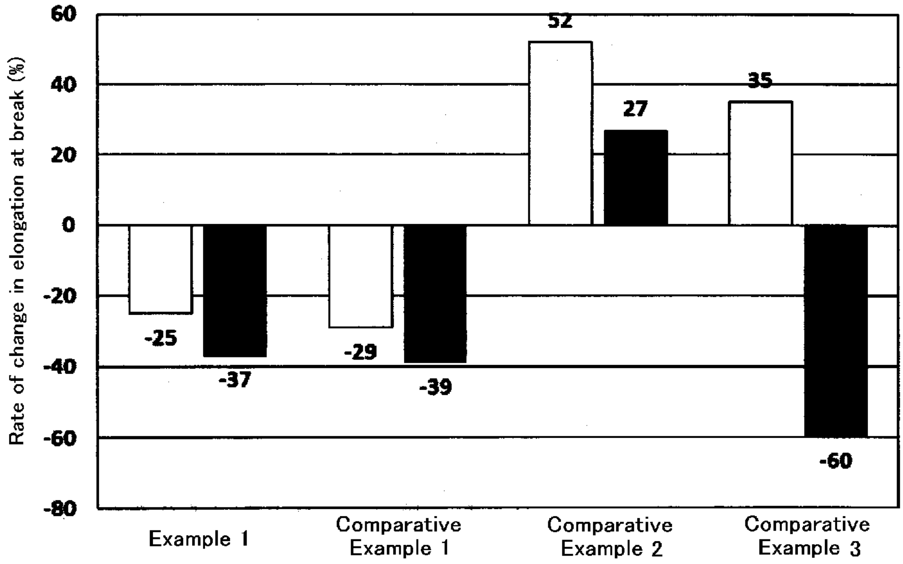
60
40
20
0
-20
-40
-60
-80
Rate of change in elongation at break (%)
52
35
27
-25
-37
-29
-39
-60
Example 1
Comparative Example 1
Comparative Example 2
Comparative Example 3

[Fig. 6]

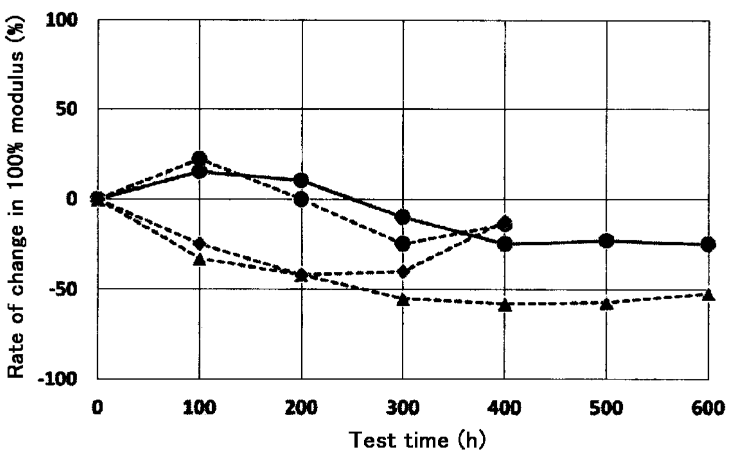
100
50
0
-50
-100
Rate of change in 100% modulus (%)
0
100
200
300
400
500
600
Test time (h)

[Fig. 7]

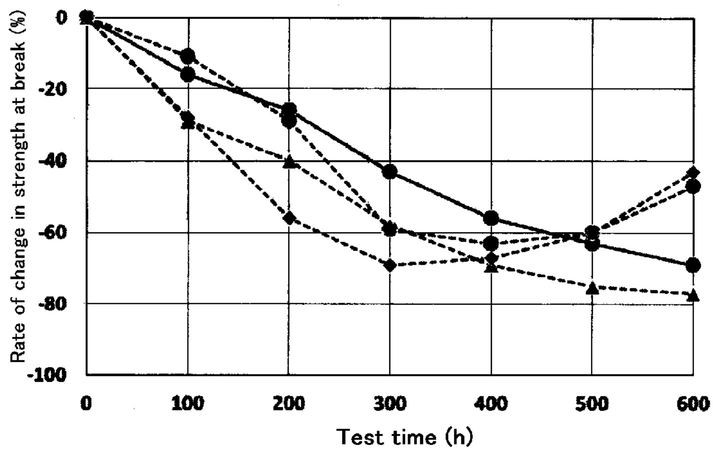
0
-20
-40
-60
-80
-100
Rate of change in strength at break (%)
0
100
200
300
400
500
600
Test time (h)

[Fig.8]

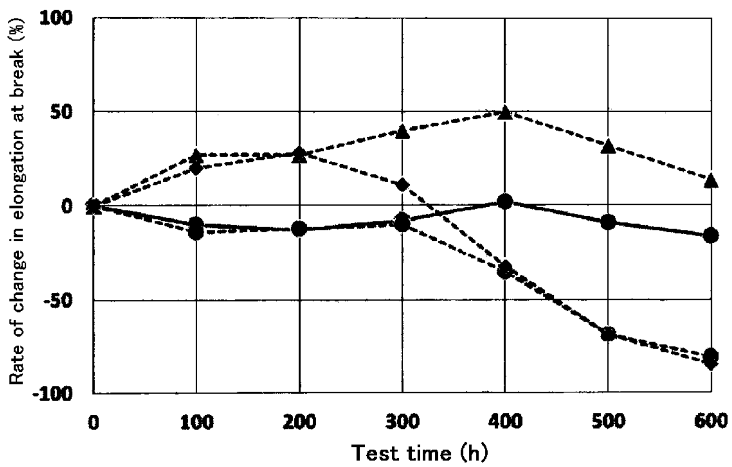
100
50
0
-50
-100
Rate of change in elongation at break (%)
0
100
200
300
400
500
600
Test time (h)

[Fig. 9]

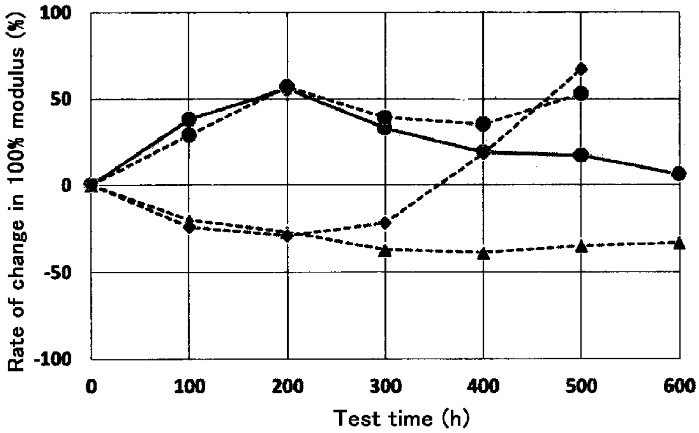
Rate of change in 100% modulus (%)
100
50
0
-50
-100
0
100
200
300
400
500
600
Test time (h)

[Fig. 10]

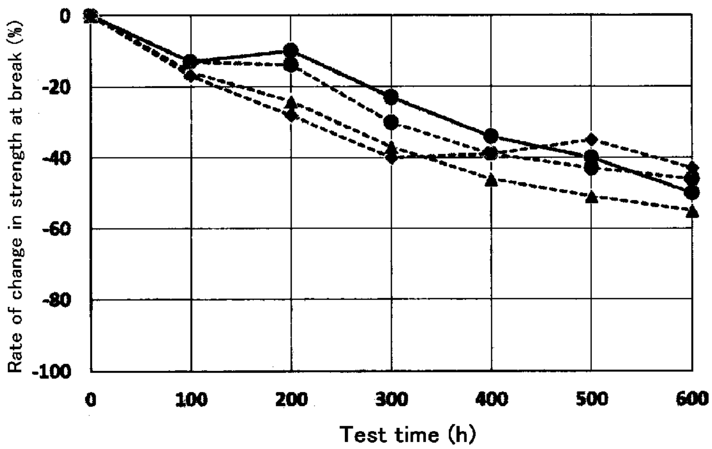
Rate of change in strength at break (%)
0
-20
-40
-60
-80
-100
0
100
200
300
400
500
600
Test time (h)

[Fig. 11]

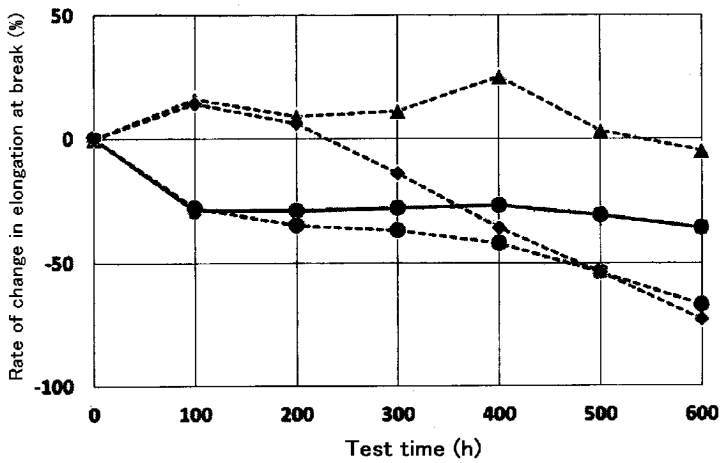
50
0
-50
-100
Rate of change in elongation at break (%)
0
100
200
300
400
500
600
Test time (h)

[Fig. 12]

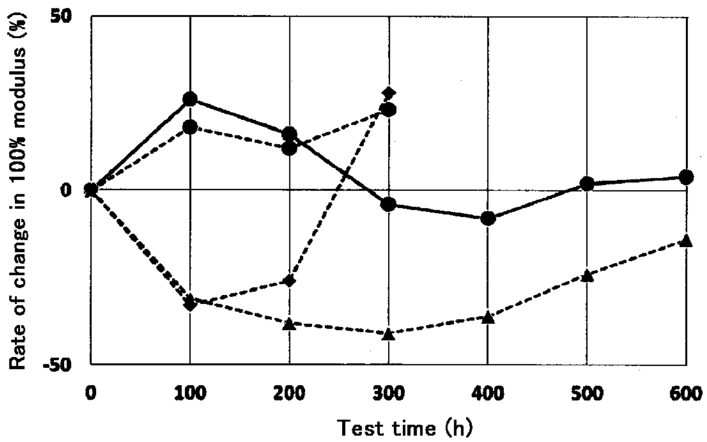
50
0
-50
Rate of change in 100% modulus (%)
0
100
200
300
400
500
600
Test time (h)

[Fig. 13]

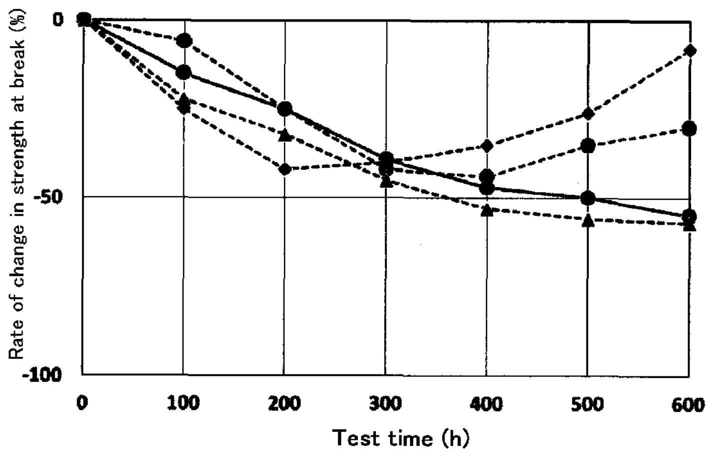
0
-50
-100
Rate of change in strength at break (%)
0
100
200
300
400
500
600
Test time (h)

[Fig. 14]

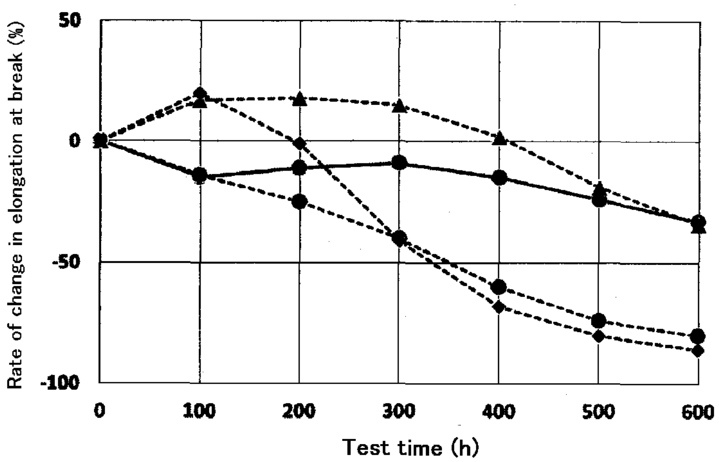
50
0
-50
-100
Rate of change in elongation at break (%)
0
100
200
300
400
500
600
Test time (h)

[Fig. 15]

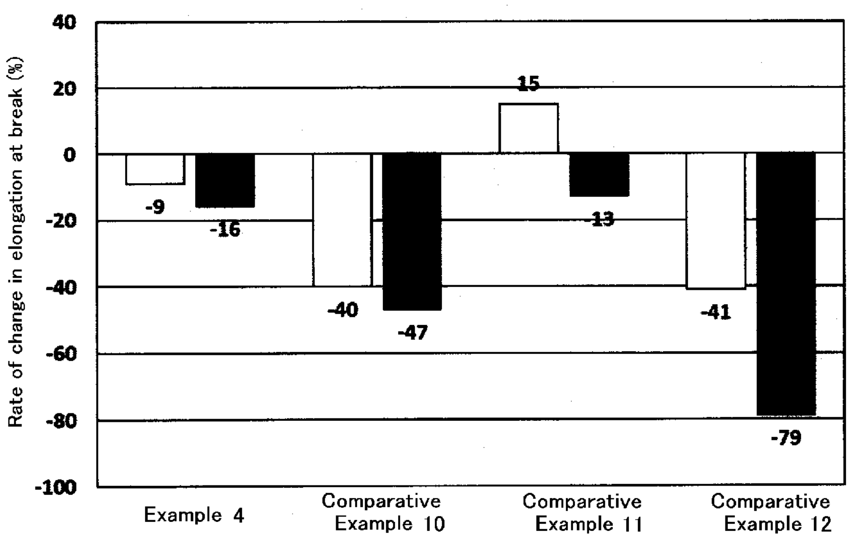
40
20
0
-20
-40
-60
-80
-100
Rate of change in elongation at break (%)
15
-9
-16
-13
-40
-47
-41
-79
Example 4
Comparative Example 10
Comparative Example 11
Comparative Example 12

## INTERNATIONAL SEARCH REPORT

International application No.
**PCT/JP2023/009304**

### A. CLASSIFICATION OF SUBJECT MATTER

***C08L 33/08***(2006.01)i; ***C08F 220/06***(2006.01)i; ***C08F 220/18***(2006.01)i; ***C08K 5/29***(2006.01)i; ***C08K 5/41***(2006.01)i
FI: C08L33/08; C08K5/41; C08K5/29; C08F220/18; C08F220/06

According to International Patent Classification (IPC) or to both national classification and IPC

### B. FIELDS SEARCHED

Minimum documentation searched (classification system followed by classification symbols)

C08L33/08; C08F220/06; C08F220/18; C08K5/29; C08K5/41

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| A | JP 2020-111551 A (UNIMATEC COMPANY, LIMITED) 27 July 2020 (2020-07-27) | 1-6 |
| A | JP 2020-111552 A (UNIMATEC COMPANY, LIMITED) 27 July 2020 (2020-07-27) | 1-6 |
| A | JP 2020-111705 A (UNIMATEC COMPANY, LIMITED) 27 July 2020 (2020-07-27) | 1-6 |
| A | JP 2011-32390 A (SEIKO KAGAKU KABUSHIKI KAISHA) 17 February 2011 (2011-02-17) | 1-6 |
| A | JP 2015-137323 A (NIPPON ZEON COMPANY, LIMITED) 30 July 2015 (2015-07-30) | 1-6 |
| A | CN 113444207 A (NANJING UNIVERSITY OF POSTS AND TELECOMMUNICATIONS) 28 September 2021 (2021-09-28) | 1-6 |
| P, A | JP 2022-185177 A (UNIMATEC COMPANY, LIMITED) 14 December 2022 (2022-12-14) | 1-6 |
| P, A | WO 2023/021832 A1 (UNIMATEC COMPANY, LIMITED) 23 February 2023 (2023-02-23) | 1-6 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 May 2023** | **23 May 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/009304**

| Patent document cited in search report | Publication date (day/month/year) | Patent family member(s) | Publication date (day/month/year) |
|---|---|---|---|
| JP 2020-111551 A | 27 July 2020 | (Family: none) | |
| JP 2020-111552 A | 27 July 2020 | (Family: none) | |
| JP 2020-111705 A | 27 July 2020 | (Family: none) | |
| JP 2011-32390 A | 17 February 2011 | (Family: none) | |
| JP 2015-137323 A | 30 July 2015 | (Family: none) | |
| CN 113444207 A | 28 September 2021 | (Family: none) | |
| JP 2022-185177 A | 14 December 2022 | (Family: none) | |
| WO 2023/021832 A1 | 23 February 2023 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11021411 A
- WO 201158918 A1
- JP 2010254579 A
- WO 2006001299 A1
- JP 2015227402 A
- WO 2011093443 A1
- JP 2009209268 A
- JP 4264106 A
- JP 5230132 A
- WO 2020158132 A1
- JP 2009084514 A
- JP 2020111552 A


### Non-patent literature cited in the description


- *Rubber Chem.Technol.*, 1973, vol. 46, 106
- *Rubber Chem.Technol.*, 1979, vol. 52, 883